# EUROPEAN PATENT SPECIFICATION

(11) **EP 1 608 236 B1**
(45) Date of publication and mention of the grant of the patent: **05.11.2014**
(21) Application number: 04749730.0
(22) Date of filing: 02.04.2004
(51) Int. Cl.: A23L 1/30, C12P 19/14, A61P 3/06

(54) **IMPROVED DIETARY FIBER CONTAINING MATERIALS COMPRISING LOW MOLECULAR WEIGHT GLUCAN**
VERBESSERTE DIÄTFASER, DIE NIEDERMOLEKULARES GLUCAN UMFASSENDE MATERIALIEN ENTHÄLT
FIBRE DIETETIQUE AMELIOREE CONTENANT DES MATIERES COMPRENANT DU GLUCANE DE FAIBLE POIDS MOLECULAIRE

(30) Priority: 02.04.2003 US 460758 P
(43) Date of publication of application: 28.12.2005
(73) Proprietor: CARGILL, INCORPORATED, Wayzata, Minnesota 55391 (US)
(72) Inventor: ZHENG, Guo-Hua, Centerville, OH 45459 (US); HESS, Richard, Chanhassen, MN 55317 (US); KHARE, Anil, Crystal, MN 55429 (US); HILBERT, Brent, South Haven, MN 55382 (US); DEGUISE, Matt, Denver, Colorado 80202 (US)
(74) Representative: Wibbelmann, Jobst
(86) International application number: PCT/US2004/010355
(87) International publication number: WO 2004/086878

(56) References cited:
- EP-A- 1 208 752
- US-A- 4 110 163
- US-A- 4 247 636
- US-A- 4 448 790
- US-A- 5 190 755
- US-A- 5 458 893
- US-A- 6 143 883
- US-A1- 2003 154 974
- DATABASE MEDLINE US NATIONAL LIBRARY OF MEDICINE (NLM), BETHESDA, MD, US; Federal register 2002; 67 (191): 61773-61783 2002, "Foodlabeling: health claims; soluble dietary fiber from certain foods and coronary heart disease. Interim final rule" XP002297797 Database accession no. NLM12361061

## Description

### FILED OF THE INVENTION

The invention relates to dietary fiber compositions and processes for making such compositions. The invention also relates to food and beverage products containing the dietary fiber compositions.

### BACKGROUND

β-glucan is a polysaccharide typically found in cereal grains. It comprises linear polymers of β-glucosyl residues, which are polymerized through (1->3) and (1->4) linkages in varying proportions. The weight average molecular weight of isolated oat and barley β-glucan has been reported to be on the order of 1,000,000 daltons-though the molecular weight of cereal β-glucan has not been reported.

Compared to other cell wall components such as cellulose and lignin, β-glucan is highly soluble in water. However, when ingested, the soluble β-glucan is not hydrolyzed in the small intestine of human digestive systems. Hence, β-glucan is classified as a soluble dietary fiber. Many studies have shown that β-glucan soluble fiber reduces serum cholesterol, regulates glycemic response, and enhances the growth of bifidobacteria. Physiological properties such as these lead to health benefits such as lowering the risk of cardiovascular and intestinal diseases, enhancing immune activity, and promoting regularity. Recent reports have concluded that foods containing at least 3g/day or 0.75g/serving of β-glucan from oats and oat products may have the health benefit of reducing the risk of cardiovascular disease in humans see e.g. Federal Register (USA) of 2 oct. 2002, vol 67, Nr 191, pages 61773- 61783.

The richest source of β-glucan in human diets is cereal grains. Almost all cereals have been reported to contain β-glucan. The concentration of β-glucan is higher in barley and oats, typically ranging from 2 to 14%, but lower (less than 2%) in other cereal grains.

For example, it will require at least a 12 gram serving of oat bran to provide 0.75 grams of β-glucan. The low concentration of β-glucan in cereal grains has diminished the attractiveness of β-glucan as a commercial food item. Hence, there is need for processes allowing the forming of dietary fiber compositions having a high concentration of β-glucan.

Purified fractions of hydrolysed β-glucan are disclosed in e.g., US 5458893 and US 4110163.

### SUMMARY OF THE INVENTION

The invention is defined by the claims and relates to dietary fiber compositions containing β-glucan, methods of making such compositions, food or beverage products containing such compositions, and therapeutic products containing such compositions. The dietary fiber compositions have a high β-glucan content of about 60% by weight or more. The word "about" is used to account for variance in measurement due to inherent errors associated with measurement techniques. The word "about", even if not explicitly used, is understood to modify all measurements disclosed, unless otherwise stated. As well, the dietary fiber compositions according to the invention can also have one or more properties considered favorable for food ingredients. For example, the dietary fiber compositions can have one or more of the following properties: a low weight average molecular weight; a low viscosity; a low protein content; a low fat content; high stability in water; bland flavor. Dietary fiber compositions according to the invention can also have neutral mouthfeel. Consequently, dietary fiber compositions according to the invention can be used to enhance the nutritional content of ice cream, yogurt, baked goods, bars, beverages, or certain other foods-without affecting, or with little impact to, the taste or other sensory attributes of the food. Dietary fiber compositions also can be used to provide certain therapeutic benefits.

The invention provides dietary fiber compositions, which are isolated from a cereal grain containing β-glucan. The dietary fiber compositions include at least 60% by weight of a β-glucan compound that is a modified form of the cereal β-glucan, from which the composition is derived. The modified β-glucan has a weight average molecular weight ranging from about 120 kDa to about 250 kDa. The dietary fiber composition has a low viscosity. "Low viscosity" is understood to mean, from hereonin, a viscosity, as measured in accordance with the protocol described later in Example 10, that is sufficiently low that the dietary food composition can have utility as a food ingredient over a wide range of products. For example, a viscosity of 100 cps or less can be considered low. According to some embodiments, the dietary fiber compositions can have a bland flavor. "Bland flavor", as understood from hereonin, is associated with a flavor intensity score of about 5 or less as determined by a standardized sensory evaluation (described in Example 23). The dietary fiber compositions are highly stable in water. By "highly stable in water," it is meant that a 1% by weight solution of the dietary fiber composition in water shows little to no precipitate, when stored overnight (16 hours) at refrigeration temperature 4.4°C (40°F). The protocol for forming the initial solution may vary depending on the particular dietary fiber. For example, some dietary fibers may form a solution when spoon-stirred into water at room temperature. Other dietary fibers, however, may require the use of a powered mixer and heated water to form a solution.

The invention also provides food and beverage products, and/or therapeutic products that contain dietary fiber compositions, which include at least one β-glucan compound that is a modified form of the cereal β-glucan according to claim 1. The products can include, as non-limiting examples, baked goods, cereals, extruded snacks, meat substitutes, bars, salad dressings, soups, sauces, yogurts, frozen desserts, refrigerated and frozen doughs, and confections.

The invention also provides methods of making dietary fiber compositions according to claim 1, which include at least one β-glucan compound that is a modified form of the cereal β-glucan. In some embodiments, the process includes using an enzyme or combination of enzymes to perform a non-specific digestion of polysaccharides found in a cereal. The polysaccharides include β-glucan and starch, and the digestion reduces the weight average molecular weight of the β-glucan and breaks down the starch. In some embodiments, a second digestion is performed to further break down the starch.

One aspect of the invention relates to a method for obtaining a dietary fiber containing material according to claim 1. The method comprises: (1) forming an aqueous mixture having components which comprise a first exogenous enzyme, a second exogenous enzyme, and one or more cereal grains; wherein the one or more cereal grains comprise β-glucan and starch; (2) cleaving by a first hydrolysis reaction catalyzed by the first exogenous enzyme at least some of the bonds of the β-glucan, wherein the average molecular weight of the β-glucan is reduced; and cleaving by a second hydrolysis reaction catalyzed by the second exogenous enzyme at least some of the bonds of the starch; wherein at least a portion of the first hydrolysis reaction and a portion of the second hydrolysis reaction occur substantially simultaneously; (3) raising the temperature of the aqueous mixture to a level sufficiently high to substantially inactivate the first exogenous enzyme; and adding to the aqueous mixture a third exogenous enzyme; (4) cleaving by a third hydrolysis reaction catalyzed by at least the third exogenous enzyme at least some of the remaining uncleaved bonds of the starch, wherein the starch is substantially digested and the third exogenous enzyme can be the same as or different from the second exogenous enzyme; (5) separating and isolating a portion of the mixture, wherein the separated portion contains at least some of the β-glucan; (6) purifying the β-glucan within the separated portion; and (7) obtaining a dietary fiber containing material; wherein the dietary fiber containing material comprises at least 60 percent β-glucan; and the average molecular weight of the β-glucan within the dietary fiber containing material is of 120 k Da to 250 k Da.

Another aspect of the invention relates to another method for obtaining a dietary fiber containing material. The method comprises: (1) forming an aqueous mixture having components which comprise a first exogenous enzyme and one or more cereal grains; wherein the one or more cereal grains comprise β-glucan and starch; (2) cleaving by a first hydrolysis reaction catalyzed by the first exogenous enzyme at least some of the bonds of the β-glucan, wherein the average molecular weight of the β-glucan is reduced; (3) raising the temperature of the aqueous mixture to a level sufficiently high to substantially inactivate the first exogenous enzyme; (4) adding to the aqueous mixture additional enzyme material, wherein the additional enzyme material comprises a second exogenous enzyme; (5) cleaving by a second hydrolysis reaction catalyzed by the second exogenous enzyme at least some of the bonds of the starch; (6) separating and isolating a portion of the mixture, wherein the separated portion contains at least some of the β-glucan; (7) purifying the β-glucan within the separated portion; (8) obtaining a dietary fiber containing material; wherein the dietary fiber containing material comprises greater than 40 percent β-glucan; and the average molecular weight of the β-glucan within the dietary fiber containing material is of 120 k Da to 250 k Da.

In related aspects of the invention, the hydrolysis reactions described in the preceding paragraphs occur within certain temperature ranges. In other related aspects of the invention, the dietary fiber containing material and the therapeutic composition can have certain excellent properties, such as neutral mouthfeel, high quantity of dietary fiber, low fat content, low protein content, high whiteness, high aqueous solubility, and high dry flowability. In still other related aspects of the invention, the dietary fiber and the therapeutic composition have a particular molecular weight distribution and a particular polydispersity.

A further aspect of the invention relates to a food product comprising β-glucan, as defined in claim 1, and wherein the food product has a neutral, non-lubricious mouthfeel.

Still further aspects of the invention relate to the dietary fiber materials, the therapeutic compositions, and the β-glucan having modified molecular weight made by the various methods described herein.

An even further aspect of the invention relates to methods of using the therapeutic compositions of the invention for hypochloesterolemic applications and other therapeutic applications.

### BRIEF DESCRIPTION OF THE DRAWINGS

**FIG. 1**-Figure depicting molecular weight distribution of Beta-glucan of the invention for samples of average Mw of about 120,000 and average Mw of about 170,000.
**FIG. 2**-The effect of the dietary fiber containing composition of the invention on average total cholesterol levels for six weeks consumption for F1 male hamsters. For each group, n=10.
**FIG. 3**-The effect of the dietary fiber containing composition of the invention on average HDL cholesterol levels for six weeks consumption for F1 male hamsters. For each group, n=10.
**FIG. 4**-The effect of the dietary fiber containing composition of the invention on average non-HDL cholesterol levels for six weeks consumption for F1 male hamsters. For each group, n=10.

### DETAILED DESCRIPTION OF THE INVENTION

### Abbreviations

- **cp**: centipoise
- **deg C**: degrees Centigrade
- **deg F**: degrees Fahrenheit
- **g**: gram
- **kg**: kilogram
- **hr**: hour
- **kDa**: kiloDalton
- **l**: liter
- **min**: minute
- **ml**: milliliter
- **Mn**: molecular number
- **Mw**: molecular weight
- **W/W**: a comparison of the quantity of two materials on a weight to weight basis
- **V/V**: a comparison of the quantity of two materials on a volume to volume basis
- **%**: percent; percent is described on a W/W basis unless otherwise indicated

The invention relates to a dietary fiber composition having a β-glucan content as defined in claim 1. The dietary fiber composition can have excellent physicochemical, physiological, and sensory properties. For example, the composition can have low molecular weight, a particular molecular weight distribution, and a particular polydispersity. In addition, the composition can have excellent food ingredient properties. For example, the dietary fiber composition has a low viscosity, of less than or equal to about 100 cps, less than or equal to about 55 cps, less than or equal to about 5 cps. In some embodiments, the viscosity can range from about 20 cps to about 100 cps. . Dietary fiber compositions according to the invention can also have a low protein content, such as for example less than or equal to about 3%, less than or equal to about 2%. In some embodiments, the protein content can range from about 1% to about 3%. However, the invention also encompasses dietary fiber compositions with higher protein contents, such as for example ranging from about 7% to about 10%. Dietary fiber compositions according to the invention can also have a low fat content, such as for example less than or equal to 2%, and less than or equal to 1%. However, the invention also encompasses dietary fiber compositions with a higher fat content. As a food ingredient, the material can have high β-glucan content which provides nutrient to the body. Because the dietary fiber material can also have neutral mouthfeel, it can be used to enhance the nutritional content of ice cream, yogurt, baked goods, bars, beverages, or certain other foods-without affecting, or with little impact to, the taste or other sensory attributes of the food. As a further benefit, the dietary fiber composition can be used to provide certain therapeutic benefits, such as anticholesterol activity.

In addition to the high β-glucan fiber content, the material can also contain other dietary fiber. Thus having a high total dietary fiber content-a total level exceeding that of the β-glucan alone. Other benefits relating to the dietary fiber material can include one or more of: excellent whiteness, low fat, low protein, high aqueous solubility, and high dry flowability.

The invention also relates to methods for making the dietary fiber compositions according to claim 1. The methods comprise using an enzyme or combination of enzymes to non-specifically digest polysacharides found in a cereal grain. The method can be accomplished in one or more than one enzymolysis step. According to some embodiments, two enzymolysis steps are used and the temperature is raised at the end of the sole step, or at least between steps. According to other embodiments, the temperature is maintained at the end of the sole step or at least between the two steps. According to some embodiments, the dietary fiber composition can be obtained by using particular exogenous enzymes under certain conditions to digest cereal grains. Although the enzymes substantially hydrolyze any starch occurring in the cereal grain into small molecules-the enzymes only partially hydrolyze β-glucan molecules. The partially digested β-glucan molecules are then separated, isolated, and purified. The resultant β-glucan has reduced molecular weight, a particular molecular weight distribution, and a particular polydispersity.

The invention also provides methods for the modification of β-glucan fiber occurring in cereal grain, and for the extraction of the resultant modified β-glucan.

The starting cereal grain comprises β-glucan and starch. In an embodiment, the molecular characteristics of the β-glucan are modified using exogenous cellulases of microbial and/or plant origin at a temperature higher than the gelatinization temperature of the starch. The term cellulase and cellulases in this invention is used to refer to those enzymes that hydrolyze polymers composed of beta-glucose linkages. Such enzymes include beta-glucosidase and lichinase. Simultaneously with the β-glucan modification, the starch is at least partially hydrolyzed with an amylotic enzyme.

After the β-glucan molecular characteristics have been modified the desired amount, the cellulase is inactivated by raising its temperature above its inactivation level. Additional amylotic enzyme is then added and the hydrolysis of the starch is continued until its digestion is substantially complete.

From the processing standpoint, the invention can offer one or more of the following advantages:
- controlled modification of β-glucan molecular characteristics by varying cellulase dosages and conditions (pH, temperature, time length);
- a more effective extraction of β-glucan, resulting in higher β-glucan solubilization and ultimately higher β-glucan yield; and
- enabling of higher incorporation of starting cereal material in the process, resulting in improved processing efficiency.

In certain embodiments, it is possible to have only a small amount (or none) of an amylotic enzyme acting simultaneously with the cellulase. In such an embodiment, the cellulase partially digests the β-glucan, the temperature is then raised to inactivate the cellulase, and an amylotic enzyme is added to digest the starch.

In certain other embodiments, it is possible to have sufficient amylotic enzyme present with the cellulase, such that the addition of additional amylotic enzyme is not needed after the temperature of the mixture is raised to inactivate the cellulase. In such an embodiment, at the lower temperature, the cellulase partially digests the β-glucan and the amylotic enzyme partially digests the starch. The temperature is than raised to inactivate the cellulase. The amylotic enzyme remains active at the higher temperature and continues to digest the starch until it is substantially digested. As noted above, the present invention is not limited to processes in which a temperature increase occurs.

The β-glucan containing dietary fiber compositions produced by the present methods can have particular molecular characteristics, resulting in certain physico, physiological, and sensory properties. Without being bound by theory, it is believed that the resultant composition occurs, in part, because of the special functioning of certain exogenous cellulases at temperatures higher than the starch gelatinization temperature. Hence it may be preferable, to choose an enzyme which can be active at temperatures above the starch gelatinization temperature, such as above about 60 deg C for barley or about 67 deg C for oat.

The exogenous cellulases and amylolytic enzymes may be enzyme preparations from various origins or a single preparation from a single origin.

Certain exogenous cellulases have been tested, including Spezyme LT-75 and Spezyme LT-300 which are enzyme preparations having both cellulase activity and amylase activity. Spezyme LT-75 and Spezyme LT-300 are derived from *Bacillus amyloliquefaciens,* and are products of Genecore International. Although the inventors have not found cellulase activity reported in the literature for Spezyme LT-75 or Spezyme LT-300, the inventors have discovered through their own experiments that both Spezyme LT-75 and Spezyme LT-300 have cellulase activity. The discovery of such cellulase activity is described in Example 14.

Although Spezyme LT-75 and Spezyme LT-300 were tested, it is believed that the specific cellulase is not critical, and other enzymes may be suitable, preferably enzymes which are active above the starch gelatinization temperature, and which can non-specifically digest polysaccharides. Hence candidate cellulases would include certain enzymes from bacteria such as *Bacillus anyloliquefacients* and *Bacillus licheniformis;* certain enzymes from fungi such as *Tricodenna longibrachiatum* and *Tircoderma hamatum;* and certain enzymes from yeast such as *Saccharomyces cerevisiae* and *Candida oloephila.* Example 17 below identifies a methodology for identifying suitable enzymes.

In a similar fashion, it is believed that specific exogenous amylolytic enzymes used for the digestion of the starch are not critical. However, the enzyme should be chosen so that it is functional within the temperature range at which it is used. Hence, in view of the various embodiments described herein, candidate enzymes should be functional for at least a portion of the temperature range of about 60 deg C to about 110 deg C. Therefore candidate enzymes include certain enzymes from bacteria such as *Bacillus anyloliquefacients* and *Bacillus licheniformis*; certain enzymes from fungi such as *Aspergillus Oryzae* and *Aspergillus niger*; and certain enzymes from yeast such as *Candida tsukubaensis.* Fred L (a high temperature alpha-amylase prepared from *Bacillus licheniformis,* available from Genencor International) is a particular alpha-amylase which has been tested for the invention.

Some of the modified molecular characteristics of the β-glucan can include one or more of a particular molecular weight, particular molecular weight distribution, particular polydispersity, particular molecular shape in aqueous systems, and a particular ratio of (1->3)/(1->4)- β-linkages of glucosyl units. Some of the modified physicochemical properties can include one or more of a lower viscosity, non-gelling characteristics, and high solubility in water. Some of the physiological properties can include one or more of: cholesterol lowering effect, glycemic response modulation, enhancement of bifidobacteria growth, improvement in mineral absorption in humans and other animals. Some of the food sensory properties can include one or more of: neutral mouthfeel, lack of lubricity, bland flavor, and minimal viscosity-building or body-building effect.

The starting cereal may be any cereal grain that contains β-glucan and/or its milling fraction of plant material. Typical examples include: barley, oats, rye, triticale, wheat, rice, corn, amaranth, quinoa, millet, sorghum, and other similar cereal. The β-glucan containing materials can be either in ground form or intact form.

A typical process for β-glucan modification, extraction and starch hydrolysis comprises: (1.) This step enables β-glucan modification, extraction and partial hydrolysis of starch. The step is accomplished by treating a β-glucan containing material with exogenous cellulase and amylolytic enzyme in an aqueous system at temperatures above the starch gelatinization temperature (typically about 60 to about 90 deg C), for about 15 to about 360 min, at a pH in the range of about 3 to about 11. The β-glucan undergoes partial hydrolysis. In addition, the β-glucan achieves particular molecular characteristics, including a reduction in molecular weight, a particular molecular weight distribution, and a particular polydispersity. (2.) This step which enables control of the molecular modification of the β-glucan of the previously described step. The step enables inactivation of the exogenous cellulases when the molecular characteristics of the β-glucan have been modified to the desired characteristics. The step can be accomplished in alternate ways, such as: (2a.) heating the aqueous system to a temperature at which the cellulases are inactivated. Although the temperature will depend on the particular cellulase, the temperature is typically about 80 to about 120 deg C, (2b.) decreasing the system pH to less than 4 or increasing the system pH to greater than 9, (2c) adding enzyme inhibitors. Typical enzyme inhibitors include cellulase analogs, substrate analogs, certain salts, and other similar materials, or (2d) providing physical treatment such as sonic treatment, electrical treatment, or other similar physical treatments. (3.) This step enables the further hydrolysis of the starch molecules so they can be separated from β-glucan molecules by means, or combination of means, known to those of ordinary skill in the art. Typical means include: alcohol precipitation, salt precipitation, ultrafiltration, freeze-thaw treatment, film-forming, and other similar separation means. The step involves incorporating an amylolytic enzyme at higher temperature, typically about 80 to about 120 deg C, for about 15 to about 360 min, at pH in the range of about 4 to about 10. Unless further molecular modification of the β-glucan is desired, it is important that the temperature of this step be sufficiently high that the cellulase of step 1 remains inactive. The present step continues until the starch molecules are sufficiently digested to allow separation from the β-glucan. Should the starch molecules be sufficiently digested at the start of this step, the step may not be necessary.

A clear aqueous extract containing mainly solubilized β-glucan and hydrolyzed starch derived from the enzyme treatments as described above then can be separated from insoluble materials by means, or combination of means, known to those with ordinary skill in the art. Such means typically include: filtration, centrifugation, flotation, decanting, and other similar separation means.

The β-glucan with modified molecular characteristics contained in the clarified extract can then be separated from hydrolyzed starch, soluble protein, lipid and other minor components by means, or combination of means, known to those with ordinary skill in the art. Such means typically include: precipitation with water-miscible solvents such as alcohols and acetone, or precipitation with salts such as ammonium sulphate and calcium chloride, ultrafiltration, freeze-thaw, film-forming, and other similar means.

The separated β-glucan can be dried by means, or combination of means, known to those with ordinary skill in the art. The dry β-glucan are typically at least about 60% pure on a dry weight basis.

The separated β-glucan possesses particular molecular characteristics. Present data show average Mw ranging from about 120,000 to about 170,000. The resultant β-glucan is highly soluble in water and forms a non-viscous solution. For example, a solution containing 1% β-glucan of the invention would have a viscosity of about 1 to about 1000 cps at 25 deg C.

When formulated to food or feed, the β-glucan of the invention has certain therapeutic benefits. For example, when consumed by human or animals, cholesterol reduction, blood glucose modulation, increase in bifidobacteria growth, and mineral absorption are expected.

### Description of a preferred embodiment of the invention

1. The β-glucan containing cereal or plant materials are disintegrated (ground or milled) prior to β-glucan modification and extraction.
2. β-glucan extraction and modification.
   a. This step is preferably carried out in an aqueous slurry system at temperatures higher than the starch-gelatinization temperature with the co-existence of exogenous cellulases and exogenous amylolytic enzymes. The step achieves modification of the β-glucan molecules and at least partial hydrolysis of the starch. In this preferred embodiment, at least a portion of the modification of the β-glucan molecules and the hydrolysis of the starch occur simultaneously.
   b. Although multiple enzyme preparations can be employed, it is preferred to use a single enzyme preparation that contains both cellulase and amylase activities. Typical examples of such single enzyme preparations include Spezyme LT-75 and Spezyme LT-300 (products of Genencore International) derived from *Bacillus amyloliquefaciens*.
   c. The preferred temperature is in the range of about 60 to about 90 deg C, more preferably about 60 to about 80 deg C, and most preferably about 65 to about 75 deg C to facilitate starch hydrolysis by amylase while allowing the exogenous β-glucanase to modify the β-glucan molecules.
   d. The preferred pH is in the range of about 4 to about 10, more preferably about 5 to about 8, and most preferably about 5 to about 7.
   e. The preferred length of time for this step is about 15 to about 120 minutes, more preferably about 30 to about 120 minutes, and most preferably about 30 to about 60 minutes.
3. For control of the degree of molecular modification by cellulase, the cellulases are preferably inactivated by increasing temperature to about 80 to about 120 deg C, more preferably about 90 to about 120 deg C, and most preferably about 90 to about 110 deg C. The preferred length of time for this step is about 15 to about 120 minutes, more preferably about 30 to about 120 minutes, and most preferably about 30 to about 90 minutes.
4. For better separation of modified β-glucan from starch, the starch molecules are preferably further hydrolyzed using an amylolytic enzyme at temperature ranging from about 80 to about 120 deg C. more preferably about 90 to about 120 deg C, and most preferably about 90 to about 110 deg C. The preferred time length for this step is about 15 to about 120 minutes, more preferably about 30 to about 120 minutes, and most preferably about 30 to about 90 minutes. This step preferably occurs concurrently with the previously described step. The preferred pH is about 4 to about 10, more preferably about 5 to about 9, and most preferably about 5 to about 8.
5. The solubilized β-glucan is separated from other soluble components by means of precipitation with water miscible solvents, preferably at solvent to extract ratios (volume to volume) of about 0.2 : 1 to about 2 : 1, more preferably about 0.5 : 1 to about 2 : 1, and most preferably about 0.7 : 1 to about 1.2 : 1. Typical preferred water-miscible solvents include alcohols such as methanol, ethanol, propanol, ethylene glycol, and other similar solvents.
6. The resulting β-glucan has molecular weight in the range of about 5,000 to about 5,000,0000 daltons.
7. The resulting β-glucan has a molecular polydispersity in the range of about 1.00 to about 6.00.
8. The ratio of (1->3)/(1->4)- β-linkages of glycopyranosyl units of the resulting β-glucan is about 0.1 to about 0.9.
9. When dissolved with water, the resulting β-glucan solution exhibits viscosity in the range of about 1 to about 10,000 cps at 25 deg C.
10. When formulated in food products, the β-glucan of the present invention displays, in essence, no properties which contribute to lubricity or viscous mouthfeel of the food.
11. The β-glucan of the present invention can be used in applications including, but not limited to food, nutraceutical, pharmaceutical, feed, and cosmetics.
12. When consumed by human or animal, the β-glucan of the present invention possesses sensory and therapeutic properties including, but not limited to, neutral mouthfeel, lack of lubricity, neutral taste, ability to lower cholesterol, ability to modulate blood glucose, ability to improve mineral absorption, and ability to enhance the growth of bifidobacteria.

### Molecular characteristics of modified β-glucan

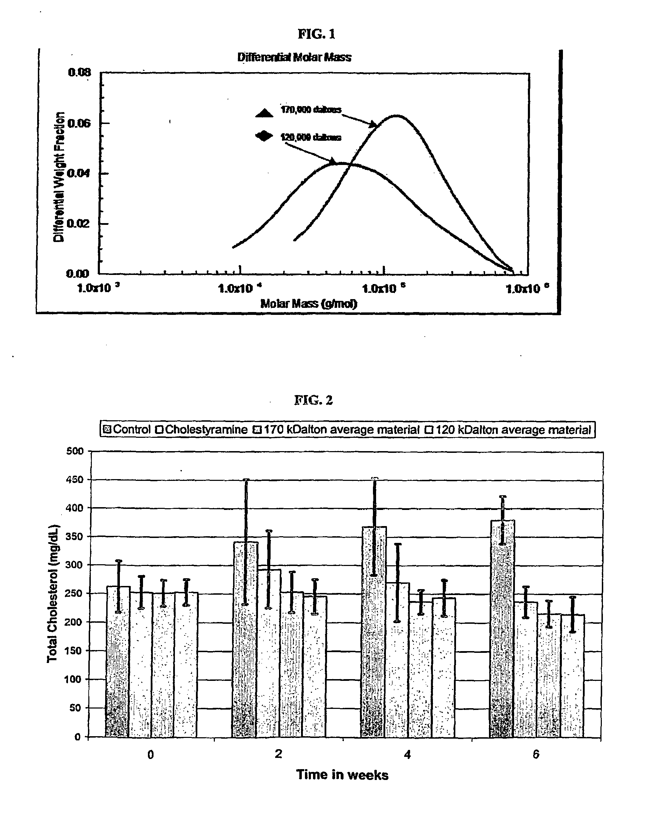

The modified β-glucan of the invention can have particular Mw characteristics. Some of the characteristics are described by FIG. 1 and illustrated in Example 8. The characteristics include a Mw in the range of 120000 to 250000 daltons with a polydispersity of Mw/Mn of about 1.00 to about 6.00.

### Inclusion of modified β-glucan into food products-sensory effects; utility as therapeutic composition

Cereal β-glucan dietary fiber, such as the dietary fiber containing material of the invention, can be incorporated into food and beverage products for fiber enrichment and the promotion of healthy cholesterol levels. Food and beverage products can include, but are not limited to, beverages, bread and baked goods, cereal, extruded snacks, meat substitutes, bars, pasta, salad dressings, soup, tortillas, and yogurt. Exemplary beverages include, but are not limited to, juice and juice drinks from fruits, vegetables, and blends; milk drinks, including fluid milks, cultured milks, fermented milks, and yogurt drinks; meal replacement beverages, such as diet and weight control beverages; powdered drink mixes; dairy-based drinks including, but not limited to, shakes, smoothies, and juice/dairy blends; dairy and non-dairy creamers; soy-based and rice-based beverages; energy and sport drinks; high protein drinks; carbonated drinks; gel drinks; water and near water; tea-based beverages and coffee-based beverages. Exemplary bars include meal replacement bars, energy bars, high protein bars, granola bars, and cereal bars with or without filling. Potential bakery applications include breads, rolls, buns, corn bread, quick breads, doughnuts, muffins, bagels, flatbreads, pancakes, waffles, cookies, cakes, pastries, croissants, scones, biscuits, crackers, pretzels, tortillas, taco shells, pasta, pie crusts, pizza crust, and bakery mixes. Examples 11 and 18-22 illustrate food and beverage products incorporating dietary fiber compositions in accordance with the present invention.

Products, such as these, which incorporate β-glucan are known to provide nutrient value in view of the glucose content of the β-glucan. In addition, the products are known to provide certain therapeutic benefits, such as the promotion of healthy cholesterol levels, modulation of blood glucose levels, improvement of mineral absorption, and enhancement of the growth of bifidobacteria. Examples 13 and 16 illustrate the ability of the dietary fiber containing material of the invention to be used as a dietary fiber therapeutic composition to promote healthy cholesterol levels. In addition to promoting healthy cholesterol levels, it is believed that the dietary fiber containing material of the invention can provide other therapeutic benefits, such as those listed immediately above.

Although β-glucan dietary fiber is often considered a good fat mimetic because it commonly displays sensory properties involving lubricity, slipperiness, or sliminess, the β-glucan dietary fiber of the present invention can has neutral sensory properties. For example, as indicated in Example 12, the β-glucan dietary fiber containing material of the invention was added to cereal bars and yogurt, and was tested in focus groups for effect on lubricity-a sensory attribute important for mouthfeel of the product. As illustrated by Example 12, the β-glucan dietary fiber containing material of the invention displayed in essence a neutral effect on lubricity.

### EXAMPLES

### Example 1

Example 1 provides a two-enzyme large-scale process in accordance with an embodiment of the invention. First, the chosen grain is cleaned and de-stoned before going through milling. The grain is milled into powder (flour) with a particle range of from about 25 microns to about 500 microns. The particle size is not critical, and thus, for example, an even smaller particle size may be usable. If the flour is to be exposed to heat and moisture, for a period of time longer than one week, the flour should be stored in dry, cool or cold room. An air-conditioned (∼25°C) environment should be sufficient for short-term storage. Otherwise, the whole grain, by itself, is stable.

A solution of hot water (65° C to 68° C) and "SPEZYME LT-75 (Genencor International)" (an enzyme which can non-specifically digest polysaccharides) is made at a ratio of 2250 liters of solution to 500 milliliter of enzyme. Before the enzyme is charged, a 25 kg of flour is charged to the water (2250 kg) to buffer the water before the enzyme is added. This buffer flour is included in the total flour charge. Thus for a hot water volume of 4500 liters, 1.0 liters of the "SPEZYME LT-75 (Genencor International)" would be charged and mixed. The milled "flour" is charged at a ratio of 5 kg of flour to 45 kgs/liters of the water solution. Thus, for a water charge of 4500 liters, the flour charge would be 500 kgs for a solids concentration of about 10% by weight in the final solution. (The process has been tested on a small-scale with a solids concentration ranging from about 5% to about 25%, and on a large-scale with a solids concentration up to 18%.) After the remaining flour has been charged, the solution is passed through a mixer to break up lumps of flour. Preferably, minimal shear should be used to eliminate flour lumps in the solution as higher shear may degrade product quality. "SPEZYME LT-75 (Genencor International)" is preferably added to prevent or alleviate gelling of the solution, which gelling can negatively impact a large scale process.

The solution is mixed for another 90 minutes while maintaining the temperature of the solution in the range of 65° ± 3°C. Immediately after the 90-minute hold time the batch temperature is raised to 95° C. This heat up step is preferably accomplished as quickly as possible. After the batch is above 95° C, a second enzyme, "Fred L (Genencor International)" is added. The enzyme is added at a ratio of 1.25 liters per 2250 kgs/liters added to the batch. For a batch size of 4500 liters, the enzyme charge is 2.50 liters. The solution is mixed for another 90 minutes while maintaining the temperature of the solution in the range of 95° ± 5°C. Other process information on the stream after the enzymes treatments includes a pH of 6.3 and a viscosity of 20 cP or lower.

After the two, 90-minute holds, the undissolved solids are separated from the solution using centrifuge technology. This step can average around 4 to 6 hours to conduct in a 5000-liter starting volume. A horizontal decanter centrifuge followed by a desludger type centrifuge have been used to generate the clarified solution. The Westfalia Separator AG, Model CA-225 has been used as the decanter centrifuge. The Westfalia Separator AG, Model SA14. has been used for the desludger centrifuge. In tests, the solids concentration in the clarified stream was on the order of 0.01 to 0.05 mls in 15 mls in spin down tubes. Lower amounts of solids in the clarified stream may be preferable with respect to the desired final product. Historically, for 51 batches, we have averaged about 2177 liters of clarified solution for a starting volume of 2400 liters. Preferably the time between desludging invtervals is lengthened by the centrifuge to minimize the loss of product in the heavies out stream.

The solution should kept at a temperature of 95°±5°C, if at all possible. Such higher temperatures can keep the solution viscosity low, and thus, solids can be removed more efficiently. Such higher temperatures can also help achieve as white a product as possible because as the solution cools (70° to 85°C), we have observed a pink/red tint in the clarified solution develop. The hot temperature can also inhibit microbiological growth, which is relevant for a food product.

The clarified solution, with a trace of solids as measured by "spin down" test tubes, is then charged to a hold tank. Ethanol (SDA 13, Canadian designation) is then charged to the clarified solution at a volume ratio of 1.1 ethanol to 1.0 extract for 92% pure ethanol. The target is to get the ethanol concentration in the total volume to around 50%. The ethanol and extract solution is mixed and then allowed to settle for 3 hours. This settling time allows the product to flocculent and to settle to the lower portion of the tank. The temperature of the solution has ranged from 40° to 55°C. We believe that the warmer temperature may improve product quality (color, purity); however, very cool solutions, below 40°C, may have a lower purity because other carbohydrates may come out of solution with the product.

At this point, the lower portion of the tank is sent for centrifugal separation of the "gums" from the supernatant. Historically, we have used a solid bowl centrifuge, a "peeler" centrifuge to recover the gums from the solution. The peeler centrifuge was a Krauss Maffei Aktiengesellschaft, Model HZ 80/1,3 SiD. The feed rate to the peeler should be controlled to avoid losses of gum when the bowl fills (so the "lights out" stream monitoring is preferable). In one embodiment, the product is recovered in the "lights out" stream by allowing the material to hold for 24 to 48 hours and form a second batch of gums. However, this approach may result in lower purities (about 50%) than is possible with other approaches.

On average, from 51 precipitations, from 2000 liters of clarified solution, precipitated with 92% alcohol, the gums recovered in the peeler centrifuge weighed 91 kgs. The gums collected in the bowl tend to have a sheet rubber feel. However, the material can readily tear under handling. The gums can be mechanically discharged. The gums can be stored, but should be kept under ethanol to avoid exposure to oxygen. The gums can be stored over a weekend with ethanol with no apparent drop in product quality.

The gums are collected and mixed, at a ratio of 1 kg of gums to 3 liters of ethanol, 92% or higher, with high shear. Historically, we have used a Ross Mixer, a high-speed disperser production unit, in a small tank 300 liters. There was also an additional pump around loop on the tank to provide vertical movement of the mixture in the small tank to ensure good mixing in the tank. The ethanol is charged with the solution being kept warm to improve color of the final product. Then the gums are added quickly to the system and mixed. The contact with the fresh alcohol dehydrates the gums and the product hardens into particles.

The particles are then isolated from the ethanol solution using centrifugal separation. Historically, we used a basket centrifuge with a manual discharge of the basket. The basket was lined with a cloth to provide a filtering media. The particles are washed with fresh ethanol (92% +) during the separation in the centrifuge. The isolated particles are then transferred to the dryer. Again, the washed material can be stored for several days under nitrogen. We want to avoid the presence of oxygen, since we have seen color problems in the drying step with oxygen present. Starting with 91 kgs of gums, on average, we recovered about 48 kgs of wet cake in the basket centrifuge.

Drying is conducted under vacuum with a nitrogen purge to minimize the presence of oxygen in the dryer. Minimum oxygen content during drying is a parameter, which can affect the final product quality. Drying with the presence of air can cause the product to significantly darken. So nitrogen purging can be used for drying. The drying can be conducted between 50° to 90°C. Historically, we have a LittleFord type dryer such as a FM model unit for the drying (jacket heating with physical mixing and a bag house unit to prevent product loss to the vacuum system). Once the process indicates the drying is complete, the product is discharged from the dryer and then milled and blended as necessary. Starting with 51 kgs of wet cake, we averaged 21 kgs of final product. This product would have a moisture content of 4% or less, with an average purity, on a dry weight basis of 70% or better.

At this time, we simply mill the product so that the maximum particle size is below 250 microns. There is no lower limit to the particle size at this time.

### Example 2

Example 2 illustrates a process in which the starting material has a 25% solids content. 2625 ml of tap water and 2.4g CaCl₂•2H₂O were charged to a 5L jacketed reactor prewarmed to 65C. The liquid was agitated while 200g of Arizona Hullless Azhoul barley flour was added. 1.75 ml of Genencor LT-75 and 4.375 ml of Genencor FredL were added. 675g of additional barley flour was added. Agitation continued at 65C for 2 hours and then the temperature was raised to 95C and held for 10 minutes. The slurry was centrifuged in a bucket type centrifuge at 6000G for 30 minutes and the supernatant was decanted to create a clarified syrup. The viscosity of the syrup was 118Cp at 25C and 10 rpm on a Brookfield viscometer using a small sample adapter.

60g of the syrup was mixed slowly under continuous agitation at 25C with 60 ml of ethanol to precipitate beta-glucan gum. After settling over night, the supernatant was decanted and the gum was centrifuged at 500G for 5 minutes. The pellet was recovered and disrupted with shear using a homogenizer in 50 ml of ethanol. The dehydrated fiber was collected by vacuum filtration and dried. The final fiber product was 63% beta-glucan on a dry weight basis.

60g of syrup that had been created by diluting 60g of the original syrup with 40g of water was precipitated under the same conditions as above. The resulting fiber product was 70.2% beta-glucan on a dry weight basis.

### Example 3

Example 3 illustrates a process in which the starting material has a 10% solids content. 2160 ml of tap water and 1.6g CaCl₂•2H₂O charged to a 5L jacketed reactor prewarmed to 65C. The liquid was agitated while 60g of Arizona Hullless Azhoul barley flour was added. 0.48 ml of Genencor LT-75 and 1.2 ml of Genencor FredL were added. 180g of additional barley flour was added. Agitation continued at 65C for 1.5 hours and then the temperature was raised to 95C and held for 15 minutes. The slurry was centrifuged in a bucket type centrifuge at 4000G for 20 minutes and the supernatant was decanted to create a clarified syrup

60g of syrup was mixed slowly under continuous agitation at 25C with 60 ml of ethanol to precipitate beta-glucan gum. After settling over night, the supernatant was decanted and the gum was centrifuged at 500G for 5 minutes. The pellet was recovered and disrupted with shear using a homogenizer in 50 ml of ethanol. The dehydrated fiber was collected by vacuum filtration and dried. The final fiber product was 73.43% beta-glucan on a dry weight basis. Total mass was 0.614g.

1825 ml of was concentrated by ultrafiltration to 975 ml on a Millipore UF unit using a 0.5m² polyether sulfone cartridge with a 10kDa molecular weight cutoff resulting in Syrup 2. 60g of syrup 2 was mixed slowly under continuous agitation at 25C with 60 ml of ethanol to precipitate beta-glucan gum. After settling over night, the supernatant was decanted and the gum was centrifuged at 500G for 5 minutes. The pellet was recovered and disrupted with shear using a homogenizer in 50 ml of ethanol. The dehydrated fiber was collected by vacuum filtration and dried. The final fiber product was 79.05% beta-glucan on a dry weight basis. Total mass was 0.943g.

### Example 4

Example 4 illustrates a process for modifying the molecular characteristics of β-glucan and separating the modified β-glucan from cereal.

Azhul hulless barley was ground in a hammer mill to pass through 5/64" screen followed by (1.5)/64" screen. 90 kg city water was heated to 70 deg C, and 15 ml of Spezyme LT75 (an enzyme preparation from *Bacillus amyloliquefaciens,* containing alpha-amylase and beta-cellulase, available from Genencor International) was added. 10 kg of the ground barley flour was slowly added to the enzyme-water mixture with vigorous agitation followed by 200 ml of 1M NaOH to adjust the mixture to pH of 5.5 to 6.5. The mixture was allowed to be agitated at 70 deg C for 30 minutes, heated to greater than 95 deg C within 15 minutes. Then 15 ml of Fred L (a high temperature alpha-amylase prepared from *Bacillus licheniformis,* available from Genencor International) was added. The mixture was allowed an additional 30 minutes agitation at 95 to 105 deg C for β-glucan extraction and complete starch hydrolysis. The hot mixture was centrifuged twice in a solid bowl centrifuge at a centrifugal force of about 4000 grams. 10 kilograms of clear crude extract was collected in 5 gallon containers, and allowed to cool to room temperature of less than 60 deg C. Then 7 liters of a denatured ethanol containing 4.25% (w/w) ethyl acetate, 6% (w/w) water, and the balance (about 90% (w/w)) ethanol was slowly added with vigorous agitation. An off-white precipitation was observed during ethanol addition. The ethanol extract mixture was allowed to settle at room temperature for 20 hours and a gel-like β-glucan cake was formed. The supernatant was carefully decanted, the cake was centrifuged at about 4000 grams, and the wet weight was then recorded. The wet cake was mixed with ethanol (600 milliliters ethanol to 200 grams wet cake) in a Waring blender for about 15 seconds, filtered through filter paper, and dried in a forced-air oven at 90 deg C for 30 minutes. The dry weight was recorded. From the original 10 kg barley flour, 5.4 kg wet cake β-glucan and 0.78 kg dry cake β-glucan were yielded. β-glucan concentration in the dry β-glucan products was about 70% when analyzed by the ADAC 995.16 standard method. When solubilized with water at room temperature, the β-glucan product produced a solution with much less viscosity than that produced without Spezyme LT-75. Typical viscosity of a 0.5% (as-is) solution was about 10 cps at 25 deg C.

### Example 5

Example 5 illustrates a process for producing a dietary fiber containing material comprising β-glucan. The average Mw of the resultant β-glucan was about 170,000 dalton.

The 170,000 dalton material of this example was produced by conducting five consecutive large scale batches. The five batches were produced by following the same procedural steps to maintain uniform batch values and practices among the five batches.

Each batch started with the weighing out of 25 and 225 kilograms (kg) of barley flour. Then 2250 liters of water, at a temperature around 65°C, were charged to the raw material charge tank. With the water around 65°C, the weighed out 25 kgs of flour was added to the charge tank. After mixing the water and flour for five minutes, 225 kgs of flour were charged along with 750 milliliters (mL) of Spezyme LT 75 (Genencor International) to the mixture in the charge tank. This mixture was then transferred to a jacketed reaction vessel through an in-line solid-liquid blender.

The mixture was then held in the jacketed reaction vessel for 30 minutes at a temperature about 65°C. At the end of the 30 minutes, another 500 ml of Spezyme LT 75 (Genencor International) was charged to the mixture in the jacketed reaction vessel. The flour/water mixture was held in the jacketed reaction vessel for another 60 minutes at a temperature about 65°C. Immediately after completing the one hour hold time, steam was applied to the jacketed reaction vessel to raise the mixture temperature to about 100°C. Once the temperature of the mixture was around 100°C, 1.10 liters of Spezyme Fred-L (Genencor International) was charged to the mixture. The mixture was held around 100°C for another 90 minutes. After 90 minutes, the mixture was then sent to centrifuges to remove the solids from the reaction mixture.

After removal of the solids from the reaction mixture, the clarified extract was sent to a hold tank. As the clarified extract was collected in the hold tank, alcohol (92% ethanol by weight) was added to the hold tank extract with mixing. After 400 liters of clarified extract was processed through the centrifuges, 400 liters of alcohol was added to the hold tank. After another 500 liters of clarified extract was processed, another 500 liters of alcohol was added to the hold tank. After all of the clarified extract was centrifuge processed, a final addition of alcohol was added to the hold tank to bring the volume of alcohol to 1.1 times of the volume of the clarified extract processed. After the final addition of alcohol, the mixture was agitated for another five minutes and then the mixture was allowed to settle for three hours.

After the three hour hold time, the clear supernatant solution in the hold tank was decanted. The cloudy mixture in the lower portion of the hold tank is sent to a peeler centrifuge to isolate the product gums. The peeler centrifuge isolated a total of 376.1 kgs of gums. The gums collected from the solid bowl centrifuge are then blended with 3 liters of alcohol for every kilogram of gums in a tank with a high speed blender. The dehydrated gums were then collected in a basket centrifuge and washed with fresh alcohol. The wet solids, a total of 164.8 kgs, were collected and dried in an agitated vacuum dryer. The solids were blended and milled and then analyzed for composition and molecular weight. A total of approximately 78.1 kgs of dried product was isolated and packaged for further study.

### Example 6

Example 6 illustrates a process for producing a dietary fiber containing material comprising β-glucan. The average Mw of the resultant β-glucan was about 120,000 dalton.

The 120,000 dalton material was produced by conducting five consecutive large scale batches. The five batches were produced by following the same procedural steps to maintain uniform batch values and practices among the five batches.

Each batch started with the weighing out of 25 and 225 kilograms (kg) of barley flour. Then 2250 liters of water, at a temperature around 65°C, were charged to the raw material charge tank. With the water around 65°C, the weighed out 25 kgs of flour was added to the charge tank. After mixing the water and flour for five minutes, 225 kgs of flour were charged along with 1250 milliliters (mL) of Spezyme LT 75 (Genencor International) to the mixture in the charge tank. This mixture was then transferred to a jacketed reaction vessel through an in-line solid-liquid blender.

The mixture was then held in the jacketed reaction vessel for 30 minutes at a temperature about 65°C. At the end of the 30 minutes, another 1250 ml of Spezyme LT 75 (Genencor International) was charged to the mixture in the jacketed reaction vessel. The flour/water mixture was held in the jacketed reaction vessel for another 60 minutes at a temperature about 65°C. Immediately after completing the one hour hold time, steam was applied to the jacketed reaction vessel to raise the mixture temperature to about 100°C. Once the temperature of the mixture was around 100°C, 1.10 liters of Spezyme Fred-L (Genencor International) was charged to the mixture. The mixture was held around 100°C for another 90 minutes. After 90 minutes, the mixture was then sent to centrifuges to remove the solids from the reaction mixture.

After removal of the solids from the reaction mixture, the clarified extract was sent to a hold tank. As the clarified extract was collected in the hold tank, alcohol (92% ethanol by weight) was added to the hold tank extract with mixing. After 400 liters of clarified extract was processed through the centrifuges, 400 liters of alcohol was added to the hold tank. After another 500 liters of clarified extract was processed, another 500 liters of alcohol was added to the hold tank. After all of the clarified extract was centrifuge processed, a final addition of alcohol was added to the hold tank to bring the volume of alcohol to 1.1 times of the volume of the clarified extract processed. After the final addition of alcohol, the mixture was agitated for another five minutes and then the mixture was allowed to settle for three hours.

After the three hour hold time, the clear supernatant solution in the hold tank was decanted. The cloudy mixture in the lower portion of the hold tank is sent to a peeler centrifuge to isolate the product gums. The peeler centrifuge isolated a total of 422.2 kgs of gums. The gums collected from the solid bowl centrifuge are then blended with 3 liters of alcohol for every kilogram of gums in a tank with a high speed blender. The dehydrated gums were then collected in a basket centrifuge and washed with fresh alcohol. The wet solids, a total of 272.1 kgs, were collected and dried in an agitated vacuum dryer. The solids were blended and milled and then analyzed for composition and molecular weight. A total of approximately 97.7 kgs of dried product, which comprised dietary fiber containing material was isolated and packaged for further study.

### Example 7

Example 7 provides analysis of the dietary fiber containing materials resulting from Example 5 and Example 6.

The dietary fiber containing materials resulting from Example 5 and from Example 6 were first analyzed for composition. Specifically, fat content, dietary fiber content, soluble fiber content, insoluble fiber content, and protein content were measured. The composition analysis was performed using standard AOAC methods. The measurements were made by Silliker Laboratories and Medallion Laboratories.

The purity of the β-glucan within the dietary fiber containing material was also measured. The purity was measured using the standard method of AOAC 995.16. In addition, the average Mw of the β-glucan was measured using the method described in Example 8.

Results of the analysis are listed in Table 1.

**Table 1**

| **Property** | **Dietary fiber containing material from Example 5** | **Dietary fiber containing material from Example 6** |
|---|---|---|
| Average Mw of Beta-Glucan | 170,000 daltons | 120,000 daltons |
| Purity of Beta-Glucan | 74.77 | 78.28 |
| RVA Data | 55 cps | 25 cps |
| | | |

| **Nutritional - Medallion** | | |
|---|---|---|
| Total Fat (%) | 0.03 | 0.11 |
| Dietary Fiber (%) | 84.9 | 86.6 |
| Soluble Fiber (%) | 84.5 | 86.2 |
| Insoluble Fiber (%) | 0.4 | 0.4 |
| Protein (%) | 2.71 | 1.75 |
| | | |

| **Silliker Results** | | |
|---|---|---|
| Total Fat (%) | 0.21 | 0.4 |
| Dietary Fiber (%) | 84.85 | 85.75 |
| Soluble Fiber (%) | 84.85 | 85.75 |
| Insoluble Fiber (%) | <0.1 | <0.1 |
| Protein (%) | 2.04 | 1.37 |

### Example 8

Example 8 illustrates the determination of weight average molecular weight and weight average molecular weight distribution for the modified beta-glucan.

A 20 mg sample of finely milled beta-glucan (<0.25 mm) was added to a 50 mL glass test tube followed by addition of 100 microliters of 95% (v/v) ethanol. 20 mL of filtered (0.2 microns) ultra-pure water was added to the test tube with vortexing. The sample was heated for 1 hour in boiling water with occasionally mixing. The sample was filtered (0.45 microns) into a liquid chromatograph vial and is then injected. Size Exclusion Chromatography (SEC) coupled with Multi-Angle Laser Light Scattering (MALLS, Dawn EOS, Wyatt Technologies Inc.) and Refractive Index (RI, Waters 410) detectors was used to determine the weight average molecular weight distribution of the beta-glucan. 100 microliters of sample was injected onto the SEC columns (Shodex OH-pak SB-G/805/804/803) via a Waters 2690 HPLC system. The columns were run at 40 °C with a flow rate of 1.0 mL/min and a mobile phase (pre filtered, 0.1 microns) of 200-ppm sodium azide in water. The MALLS detector uses Astra Software (Version 4.73.04) with a dn/dc value for beta glucan of 0.150. A Debye plot was used to calculate the weight average molecular weight distribution.

### Example 9

Determination of the purity of the concentrated beta glucan product was accomplished using the AOAC 995.16 method (modified). The sample was passed through a 500 um sieve and then milled. 20 mg of samples was weighed into a 50 mL screw cap test tube with the addition of 200 uL of 50% (v/v) ethanol. The samples were mixed to ensure dispersion of the beta glucan. 5 mL of 20 mM (pH 6.5) sodium phosphate buffer and 4.7 mL of water was added to the test tube, followed by heating in boiling water for 2 minutes with intermittent mixing. The test tubes were allowed to cool and then 100 uL of Lichenase was added. The samples were incubated for one hour at 50 C with vortexing every 15 minutes.

The samples were removed and 20 mL of water was added. The samples were then filtered (0.45 um nylon) into a test tube. An aliquot of 100 uL of the filtered samples was added to two additional test tubes. The first test tube was used as a blank to account for any glucose present not attributed to beta glucan. To this test tube 100 uL of 50 mM (pH 4.0) sodium acetate buffer was added. To the second test tube 100 uL of B-Glucosidase was added. These samples were incubated for 10 minutes at 40 C. After 10 minutes, 3.0 mL of GOPOD dye was added followed by an additional heating at 40 C for 20 minutes. The samples were then removed from the oven and allowed to cool for 10 minutes prior to analysis. The absorbance was read at 510 nm and the beta glucan purity was calculate using the equation provided by Megazyme.

### Example 10

Determination of Viscosity of Barley Betafiber (BBF). The viscosity was determined using a Rapid Visco Analyzer Model 3 (RVA) at a 1% BBF concentration. The sample was accurately weighed and placed into a known volume of water inside the RVA cell. The RVA uses software that allows for viscosity profiles to be run that vary in propeller speed and cell temperature. The following program was used to determine the viscosity. The viscosity is taken to be the maximum value generated in the graph.

| **Time (min)** | **Temperature (Celsius)** | **Speed (rpm)** |
|---|---|---|
| 0 | 95 | 960 |
| 10 | 95 | 160 |
| 17 | 25 | 160 |
| 22 | 25 | 160 |
| 29 | 95 | 160 |
| 34 | 95 | 160 |

### Example 11

Example 11 illustrates a food application of a dietary fiber containing material. Specifically, it illustrates the inclusion of dietary fiber containing material prepared in accordance with Example 1 into bars. Potential bar applications for the dietary fiber containing material include: meal replacement bars, energy bars, high protein bars, granola bars, cereal bars with or without filling, and more. Nutritional information per 100g serving of the dietary fiber material used, as well as additional characteristics of the barley beta fiber products used, is provided in Table 2 below.

**Table 2**

| **Nutritional Information per 100g Serving Barley Betafiber** | |
|---|---|
| **Nutrient** | **Approximate Composition** |
| Calories/100g | 371.9 |
| Calories from fat/100g | 1.0 |
| Total Fat (%) | 0.1 |
| Saturated Fat (%) | ND |
| Cholesterol (mg/100g) | <1 |
| Sodium (mg/100g) | 19.3 |
| Total Carbs (%) | 90.9 |
| Dietary Fiber (%) | 79.6 |
| Soluble Fiber (%) | 78.6 |
| Insoluble Fiber (%) | 1.0 |
| Sugars (%) | 0.8 |
| Protein (%) | 2.7 |
| Vit A (IU/100g) | <100 |
| Vitamin C (mg.100g) | <2 |
| Calcium (mg/100g) | 175.9 |
| Iron (mg/100g) | 0.5 |
| Moisture (%) | 3.4 |
| Ash (%) | 2.8 |
| Beta-Glucan Weight Average Molecular Weight | 165,000 |

Due to the high purity of the product, only 1.1g of the dietary fiber containing material will deliver 0.75g beta-glucan. The following formula is for a meal replacement bar where 40% of the calories derive from carbohydrates and 30% each from protein and fat. Each 50g bar contains 0.75g of beta glucan and 6.25g soy protein.

### Preparation of Meal Replacement Bar

| **Ingredients** | **%** |
|---|---|
| Cargill Prolisse® Isolated Soy Protein | 15.7 |
| Calcium Caseinate | 8.6 |
| Whey Protein Concentrate | 7.8 |
| Gerkins Cocoa | 6.6 |
| Dietary fiber containing material of the invention | 2.7 |
| Vitamin and Mineral Premix | 1.9 |
| Cargill Hi-Grade® Salt | 0.8 |
| Cargill Isoclear®42 High Fructose Corn Syrup | 25.1 |
| Cargill Isoclear®43High Maltose Corn Syrup | 12.1 |
| Honey | 7.1 |
| Wilbur® Unsweetened Chocolate | 2.3 |
| Cargill Canola Oil | 1.9 |
| Cargill Soybean Oil | 1.9 |
| Water | 5.0 |
| Flavor | 0.5 |
| Total | 100.00 |

### Procedure:

1. Blend all dry ingredients.
2. Combine the syrups, honey, chocolate, and oils over low heat.
3. Add the water and flavoring to the syrup mixture and immediately combine with the dry ingredients.
4. Mix to form a dough.
5. Sheet to desired thickness and cut into 40g bars
6. Coat each bar with 10g Wilbur® Chocolate S-856 Coating.

### Example 12

Example 12 provides a protocol for a standardized sensory evaluation test. The test is performed using cereal bars and yogurt with and without dietary fiber compositions in accordance with the invention. The dietary fiber compositions used were the same as described in Example 11 and Table 2. The cereal bars and yogurt were evaluated for lubricity-a sensory attribute important for mouthfeel of the products.

The procedure for preparing the cereal bars included the following steps: Sugar, syrup, and peanut butter were melted together until smooth over medium-low heat. The dietary fiber containing material of the invention was stirred in, followed by cereal. The materials were mixed, and then spread into an ungreased 9 x 9 pan, cooled, and cut into samples. The samples were coded and evaluated by 14 untrained people in a focus group using a 9 point intensity scale for slimy mouthfeel. The following word anchors were used as graduations for the sliminess intensity scale: not slimy (1), trace (2), faint (3), slight (4), mild (5), moderate (6), strong (7), very strong (8), extremely slimy (9).

The results are listed in Table 3. As can be seen, the inclusion of the dietary fiber containing material of the invention had virtually no effect on the mouthfeel sensory perception of the lubricity of the cereal bars.

The procedure for preparing the yogurt included the following steps: Yoplait strawberry original yogurt and dietary fiber containing material of the invention were blended together with a hand blender until smooth and well dispersed. Samples were evaluated by 14 untrained people in a focus group using the same 9 point intensity scale for slimy mouthfeel described for the cereal bars.

The results are listed in Table 4. As can be seen, inclusion of dietary fiber containing material of the invention had virtually no effect on the perception of lubricity or slimy mouthfeel of the yogurt.

**Table 3**

| | **Cereal Bar without dietary fiber containing material** | **Cereal Bar with dietary fber containing material** |
|---|---|---|
| Sugar | 100 grams | 100 grams |
| Karo Syrup | 150 grams | 150 grams |
| Skippy Creamy Peanut Butter | 125 grams | 125 grams |
| Special K | 105 grams | 93.5 grams |
| β-glucan of the invention | --- | 11.5 grams |
| Total mass | 480 grams | 480 grams |
| Lubricity intensity scale value | 2.1 | 2.7 |

**Table 4**

| | **Yogurt without dietary fiber containing material** | **Yogurt with dietary fiber containing material** |
|---|---|---|
| Yogurt | 200.0 grams | 198.8 grams |
| β-glucan of the invention | --- | 1.2 grams |
| Total mass | 200.0 grams | 200.0 grams |
| Lubricity intensity scale value | 2.3 | 2.6 |

### Example 13

Example 13 illustrates the ability of the dietary fiber containing material of the invention to promote healthy cholesterol levels.

A group of 40 type F₁ male hamsters 8-10 weeks old (at the start of the study) were acclimated for one week. Then all animals were placed on a hypercholesterolemic diet (HCD) prepared by Research Diets. After two weeks on the HCD (Time = 0), all of the animals were bled for blood samples to establish a baseline for blood levels of total plasma cholesterol, high density lipoprotein cholesterol (HDL-C), and non-high density lipoprotein cholesterol (nonHDL-C). NonHDL-C includes very low density, intermediate density, and low density lipoprotein cholesterols.

All of the hamsters were then randomly assigned to one of four groups (N=10), each with a specific diet. One group, the no treatment control group, was kept on the HCD chow. The remaining three groups were assigned to a specific diet: 1) the HCD plus 0.5% cholestyramine as a positive control group; 2) the HCD plus 8% (dry weight basis) of dietary fiber containing material prepared in accordance with Example 5. This material was characterized by a 170 kDalton average Mw and viscosity of 55 cps; and 3) the HCD plus 8% (dry weight basis) of dietary fiber containing material prepared in accordance with Example 6. This material was characterized by a 120 kDalton average Mw, and viscosity of 25 cps. Additional nutritional information for the two dietary fiber compositions used is presented in Table 5 below.

**Table 5**

| **Nutrition Information per 100g Serving Barley Betafiber** | | |
|---|---|---|
| **Nutrient** | **Approximate Composition** | |
| | **170kDa dietary fiber composition** | **120 kDa dietary fiber composition** |
| Calories/100g | 358.0 | 366.0 |
| Calories from fat/100g | 0.0 | 1.0 |
| Total Fat (%) | 0.0 | 0.1 |
| Saturated Fat (%) | 0.0 | 0.0 |
| Cholesterol (mg/100g) | <1.00 | <1.00 |
| Sodium (mg/100g) | 18.3 | 31.9 |
| Total Carbs (%) | 87.1 | 90.0 |
| Dietary Fiber (%) | 84.9 | 86.6 |
| Soluble Fiber (%) | 84.5 | 86.2 |
| Insoluble Fiber (%) | 0.4 | 0.4 |
| Sugars (%) | 0.7 | 1.0 |
| Protein (%) | 2.7 | 1.8 |
| Vit A (IU/100g) | <100 | <100 |
| Vitamin C (mg.100g) | <1.00 | <1.00 |
| Calcium (mg/100g) | 208.0 | 229.0 |
| Iron (mg/100g) | 0.5 | 0.5 |
| Moisture (%) | 7.2 | 5.1 |
| Ash (%) | 2.5 | 2.7 |

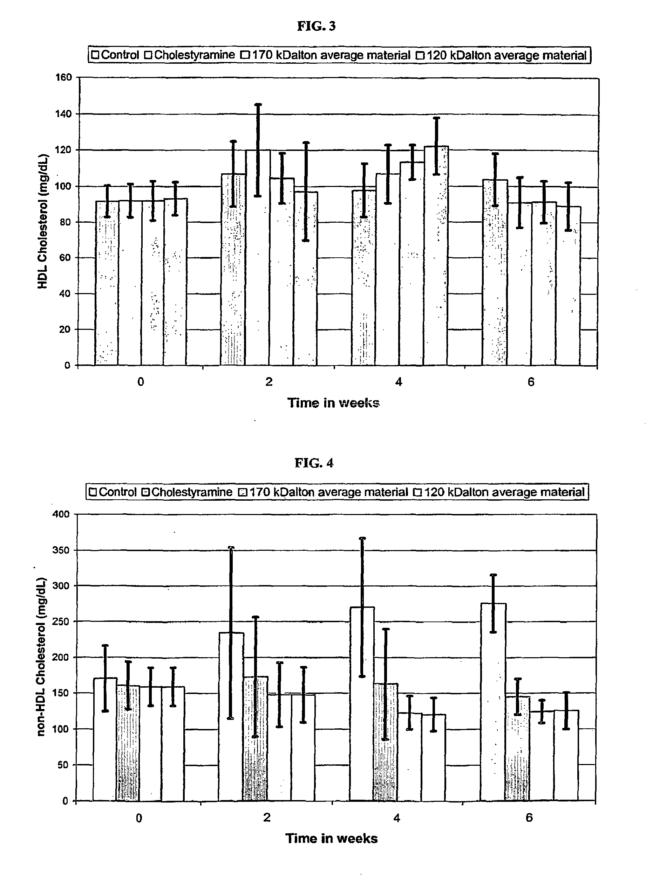

The graphs depicted in FIG 2, FIG 3, and FIG 4 illustrate the efficacy of the dietary fiber containing material of the invention, at 8% concentration in the animal chow, against the performance of the drug treatment at 0.5% cholestyramine. The dietary fiber containing material of the invention lowered the non-HDL-C values consistently equal to the drug treatment. In addition, the dietary fiber containing material did not significantly alter the average HDL-C levels for the course of the study.

### Example 14

Example 14 illustrates results which indicate that the enzyme preparations Spezyme LT-75 and Spezyme LT-300 have both cellulase and amylase activity. Spezyme LT-75 and Spezyme LT-300 are commercially available from Genencor International.

An aqueous mixture was prepared at about 70 deg C comprising about 90 kg of water and about 10 kg of ground barley. The barley comprises Beta-glucan and starch. The mixture was mixed thoroughly and allowed to stand for about 30 minutes. The mixture was allowed to stand for about 90 minutes more. No appreciable change was observed in the viscosity over the 30 minute period, or over the subsequent 90 minute period. The viscosity was measured using a Viscotek viscometer. It was concluded that at the conditions of the test virtually no degradation of the Beta-glucan of the barley, or the starch of the barley, had occurred.

A second aqueous mixture was prepared at about 70 deg C comprising about 90 kg of water, about 10 kg of ground barley, and about 15 ml of Spezyme LT-75. The barley comprised Beta-glucan and starch. The mixture was mixed thoroughly and allowed to stand for about 30 minutes. The mixture was then allowed to stand for about 90 minutes more. A substantial viscosity reduction was observed over the 30 minute period, and an even greater viscosity reduction was observed over the subsequent 90 minute period. The average Mw of the Beta-glucan was measured after the 90 minute period using the methods described in Example 5, and substantial reduction in average Mw had occurred. In view of the reductions in viscosity and Mw, it was concluded that substantial degradation had occurred in both the Beta-glucan of the barley and the starch of the barley. Hence, it was concluded that the Spezyme LT-75 had both cellulase activity and amylase activity. Although the product specification sheet for Spezyme LT-75 from Genencor International indicates amylase activity, it does not indicate cellulase activity. The inventors are not aware of a previous report of cellulase activity for Spezyme LT-75.

A third aqueous mixture was prepared at about 70 deg C comprising about 90 kg of water, about 10 kg of ground barley, and about 15 ml of Spezyme LT-300. The barley comprised Beta-glucan and starch. The mixture was mixed thoroughly and allowed to stand for about 30 minutes. The mixture was then allowed to stand for about 90 minutes more. A substantial viscosity reduction was observed over the 30 minute period, and an even greater viscosity reduction was observed over the subsequent 90 minute period. The average Mw of the Beta-glucan was measured after the 90 minute period using the methods described in Example 8, and substantial reduction in average Mw had occurred. In view of the reductions in viscosity and Mw, it was concluded that substantial degradation had occurred in both the Beta-glucan of the barley and the starch of the barley. Hence, it was concluded that the Spezyme LT-300 had both cellulase activity and amylase activity. Although the product specification sheet for Spezyme LT-300 from Genencor International indicates amylase activity, it does not indicate cellulase activity. The inventors are not aware of a previous report of cellulase activity for Spezyme LT-300.

### Example 15

Examplary one-enzyme process. A jacketed flask filled with tap water was heated to 65°C. Once the water reached temp, a small amount of barley flour (8.61% BG) was added. Then 0.02% enzyme (LT-75) was added to the water. Lastly, flour was added to an overall 10% concentration by weight. This mixture was then stirred for 90 minutes. The resulting solution was removed from the reaction vessel and centrifuged for 10 minutes at 10,000 rpm. The resulting supernatant was decanted and solids were discarded. The supernatant was precipitated with 1:1 Ethanol by weight and allowed to settle overnight. The resulting solids were isolated by decantation followed by centrifugation. The product was washed in ethanol, 5 times by weight, homogenized and filtered through Whatman #3. The resulting powder was dried overnight in a 60°C vacuum oven.

The color of the vacuum, dried product was a very bright white. The purity of this first trial was 77.4%, DWB. This experiment has been repeated twice under the same parameters with purities of 79.7% and 76.0%, DWB. However, it should be noted that this was also run once using deionized water and the purity only reached 18%. It is now known that requires 200ppm Calcium should be used to induce the alpha-amylase enzyme activity. As a side note, the resulting digestions range from 64-80% Beta Glucan recovery. The remained product is lost in precipitation, undigested or trapped in the moisture of the spent solids.

Due to the alpha- amylase and beta-glucanase activity of LT-75, a high purity beta glucan can be produced on a lab scale. This reduces energy costs and tank hold time, as well as enzyme costs in a production environment. Subsequent experiments have proven that this process can be run at 15% solids digestion, provided there is ample centrifuge capabilities.

### Example 16

6 men and 6 women who were known responders to beta-glucan were recruited for study. Those individuals were all hypercholesterolemic, hyperglycemic (glucose intolerant) men and women who were otherwise generally healthy. There were divided randomly into 2 groups and treated with either 5 grams/d of cereal incorporating a low molecular weight (LMW) dietary fiber composition or 5 grams/d of juice incorporating a LMW dietary fiber composition. Baseline labs (lipids and glucose) were taken before treatment began as well as body weight and baseline side effects. Patients were then told to consume the juice or cereal every day with food for 21 days. After 21 days, all labs were repeated as well as body weight, etc. Patients were told to maintain their "normal" lifestyle through out the treatment period but no specific assessment of diet was performed. The primary outcome variable was LDL-C. In the cereal group, LDL-C dropped from 158 to 135 (14.5%) and in the juice group from 165 to 144 (12.5%). Body weight remained unchanged. Blood glucose levels also dropped during treatment. GI (Gastro Intestinal side effects) was unchanged by the treatment. The dietary fiber composition incorporated into the cereal and juice provided to the participants is the same as the 170 kDa material described in Table 5, Example 13.

### Example 17

Examination of Alternative Enzymes. The following example provides an exemplary methodology for screening alternative enzymes. For the particular example, LT-300 was used as the benchmark for purity and molecular weight requirements. However, depending on the desired product and other factors, such as cost efficiencies, other benchmarks may be appropriate. Factors reviewed include the usability in bench-top production, whether purity requirements were met (alone or in combination with Fred-L), and the molecular weight profile.

### Material and Method:

Barley flour was used as the source of beta-glucan. For the particular example, the source or the barley flour was unknown, but the same lot was used for every trial. All digestions were run at 10% solids in tap water. The digestions were run in a glass-lined reactor, heated to individual requirements and internally monitored. The reactor was equipped with an air powered stirrer and a cold water condenser column. After heating the water to desired temperature, a small amount of buffering flour (∼1g per 100ml H₂O) was added. Enzyme was then added at .5%, solids basis. The remaining flour was then added. The pH was adjusted, if needed, with either 6N NaOH or 1M HCl depending on manufacture's pH specifications. Run time was 90 minutes.

| Enzyme | pH | Temp C | Ca²⁺ (ppm) |
|---|---|---|---|
| Tenase L-1200 | 6.0 | 70 | 200-400 |
| Clarase L-40000 | 5.2 | 50 | none |
| Multifresh | 4.2 | 70 | none |
| G997 | 5.7 | 95 | 30 |

After 90minutes the entire digestion was centrifuged for 10 minutes at 8000rpm (Beckman J2-21M r max=11300x g). Viscosity and dissolved solids measurements were taken on the supernatant, solids discarded. Extract was precipitated with a 1:1 (v:v) ratio of reagent alcohol (5% 2-Propanol, 90-91% 200 proof Ethanol and 4-5% Methanol). Samples were held overnight, centrifuged, decanted and then the solids fraction was washed with reagent alcohol using three times the sample volume. Samples were filtered through Whatman #4 paper and dried in vacuum oven at 70°C for no less than 1 hour.

### Results and Discussion:

Viscosity: A typical LT-300 digestion yields a supernatant with a viscosity near 15 centipoises. The Tenase L-1200 had viscosity measurements that came in well below that level. The Clarase L-40000 showed a viscosity reduction, but was still higher than the targeted range. The other two enzymes, Multifresh and G997 showed viscosity too high to measure. With this information, it was decided to forego further study of Multifresh and G997.

| Enzyme | Viscosity (cP) |
|---|---|
| Tenase L-1200 | 2.46 |
| Clarase L-40000 | 25 |
| Multifresh | N/A |
| G997 | N/A |
| Tenase and Fred-L | 6.09 |

**Dissolved Solids:** This measurement gives us an idea of the soluble fiber that was extracted during the digestion. Sugars and other carbohydrates are also extracted; only ∼15% of the dissolved solids precipitate into a product which meets purity. For reference, the LT-300 digestions typically run between 4.5 and 9% DS. As one can see, the amounts of solids extracted using Tenase were closer to the benchmark. Therefore, it was decided that Clarase would not be further studied.

| **Enzyme** | **% DS** | **Purity, DWB** |
|---|---|---|
| Tenase L-1200 | 6.36% | 42.6% |
| Clarase L-40000 | 2.36 | |
| Tenase L-1200 with Fred-L | 7.85% | 71.9% |

**Purity:** Based on the above data, only the Tenase L-1200 digestions were precipitated and analyzed for purity. Alone, the Tenase L-1200 only achieved a purity of 42.6%. However, in combination with a Fred-L digestion the purity reached 72%. Overall beta-glucan yield was very close to LT-300 bench scale yields, around 85% recovery.

**Molecular Weight:** Based on 3 trial runs the weight average molecular weight distribution of the Tenase L-1200 samples were lower then what was reported on the LT-300 samples (73,000 vs. 100-150,000 respectively) However, the MALLS chromatogram showed 2 peaks with the larger of the two being closer to 50,000 daltons. The second peak is believed to be an impurity peak. This work was performed using Size Exclusion Chromatography (SEC) coupled with Multi-Angle Laser Light Scattering Detector (MALLS) and Refractive Index (RI) detectors.

Regardless, Tenase L-1200 should produce a product with a lower average molecular weight than a product produced with LT-300. It should be noted that products can have a lower molecular weight when produced on a bench scale. For example, the weight average molecular weight of product produced with LT-300 ranged from about 100,000 to about 150,000 dalton on the bench scale, but was about 180,000 at the pilot scale.

### Example 18

A fiber and calcium fortified white bread was formulated using the dietary fiber composition described in Example 11 and Table 2. As indicated in Example 10, due to the high purity of the barley betafiber, only 1.1g of our product are needed to deliver 0.75g ß-glucan per 50g serving. A dough or batter containing barley betafiber may need just slightly more water and mixing time relative to conventional formulations. The following example is for a no-time bread formulation that delivers 0.75g barley betafiber, 3g fiber from Oliggo-Fiber™, and 200mg calcium per 50g serving.

**Fiber and Calcium Fortified White Bread**

| **Ingredients** | **%** |
|---|---|
| Cargill Artisan Bread Flour | 48.72 |
| Cargill Oliggo-Fiber™ F-97 Inulin | 5.75 |
| Vital Wheat Gluten | 3.08 |
| Cargill Barley Betafiber | 2.06 |
| Cargill All Purpose Shortening | 1.76 |
| Cargill Sugar | 1.64 |
| Cargill Hi-Grade Salt | 1.19 |
| Calcium Sulfate | 1.07 |
| Dough Conditioner | 0.82 |
| Yeast | 2.06 |
| Water | 31.85 |
| Total | 100.00 |

### Procedure:

1. Combine ingredients to create dough in an A-100 Hobart mixer with 10 quart bowl. Mix 1 minute on low speed and 10 minutes on second speed with a dough hook, or until the gluten reaches sufficient development. Dough temperature should be 29-30°C (84-86°F).
2. Divide dough into 510 gram pieces, round, and allow to rest 10 minutes.
3. Sheet, form, and place dough in lightly greased standard 20 cm (8-inch) bread pans.
4. Proof at 40°C (105° F) and 95% relative humidity for 45 minutes or until loaf rises 1.5 inches above the side of the pan.
5. Bake at 204°C (400° F) for 27 minutes.
6. Remove from the pans and cool.

### Example 19

A corn flakes breakfast cereal was prepared using the dietary fiber composition described in Example 11, Table 2. The following example is for an extruded and flaked breakfast cereal, formulated to deliver 3.0g of barley ß-glucan per 30g serving of dry cereal. It satisfies the daily level of ß-glucan required by the oat health claim in only one serving.

**Corn Flakes Breakfast Cereal**

| **Wet Mix Ingredients** | **%** |
|---|---|
| Water | 96.00 |
| Malt Syrup | 4.00 |
| Total | 100.00 |

| | |
|---|---|
| **Dry Mix Ingredients** | **%** |
| Cargill Corn Cones | 75.98 |
| Cargill Barley Betafiber | 13.79 |
| Cargill Sugar | 7.48 |
| Cargill Hi-Grade Salt | 1.76 |
| Mono- and Diglycerides | 0.88 |
| Vitamin and Mineral Premix | 0.11 |
| Total | 100.00 |

### Process:

An 80:20 ratio (w/w) of dry mix to wet mix was extruded, flaked, and toasted to a final moisture of about 2%.

### Example 20

A high purity (≥70%) ß-glucan composition, e.g. the barley betafiber composition described in Example 11 and Table 2, can be used in a variety of beverage applications for its functional and health benefits. Preliminary studies (see Examples 13 and 16) suggest the potential benefit of barley betafiber in reducing serum cholesterol levels. As a highly concentrated source of soluble dietary fiber, only 0.45% barley betafiber is needed to deliver 0.75g ß-glucan per 8oz serving. In addition to fiber fortification, barley betafiber may also be used to impart creamy mouthfeel, improve body, add viscosity, and suspend solids.

To effectively hydrate and disperse barley betafiber, the following techniques are suggested:
- Premix the barley betafiber with other dry ingredients such as sugar, maltodextrin, or starch to help separate the particles during dispersion. Pre-blending the barley betafiber in vegetable oil, corn syrup, or another nonsolvent may also be beneficial.
- Preheat the water phase to 90°C prior to dispersing the barley betafiber.
- Slowly meter the barley betafiber into the vortex of vigorously agitated hot water to thoroughly disperse. This can be achieved using high shear mixing. An aspirator apparatus such as a dispersion funnel and mixing eductor may also be useful.
- Allow the solution to hydrate and solubilize while mixing for 5-30 minutes. The required mixing time varies depending on the individual formula, process, and equipment used.
- Add the remaining beverage ingredients and adjust the pH after dispersion and hydration of the barley betafiber is complete for best solubility and stability.

Barley betafiber may be used to fortify a variety of carbonated and non-carbonated, concentrated and ready-to-drink, hot and cold beverages. Examples of such applications include juice, fruit and/or vegetable juice drinks, smoothies, meal replacements, milk, dairy and soy based drinks, sport and energy drinks, tea and coffee, creamers, water, frozen drinks, and more.

Following is the formula for a healthy and refreshing juice drink containing 0.75g barley betafiber per 8oz (240mL) serving. Various combinations of fruit juice concentrates and flavors can be blended to create your own custom blend. This approach may be used to produce other beverages fortified with barley betafiber as well.

**Barley Betafiber Juice Drink**

| **Ingredients** | **%** |
|---|---|
| Water | 96.886 |
| Cargill Barley Betafiber | 0.45 |
| Cargill Fruit Juice Concentrate | 2.00 |
| Cargill High Intensity Sweeteners | 0.22 |
| Cargill Acidulant | 0.18 |
| Flavor | 0.15 |
| Color | 0.014 |
| Potassium Citrate | 0.10 |
| Total | 100.00 |

### Procedure:

1. Heat water to 90° C.
2. Slowly sprinkle barley betafiber into the vortex of the water using high shear mixing. Mix for 15 minutes.
3. Add fruit juice concentrate, sweeteners, acidulants, flavor, and color. Mix for 5 minutes.
4. Adjust pH to 3.2 with acidulant.
5. Thermally process and fill.

### Example 21

A high purity (≥70% purity) ß-glucan product in accordance with the present invention, e.g. the barley betafiber described in Example 11 and Table 2, can be used in a variety of soup and sauce applications. As a highly concentrated source of soluble fiber, only 1.1 g of our barley betafiber are needed to deliver 0.75g barley ß-glucan per serving. Barley betafiber may also be used to impart a creamy mouthfeel, improve cling, add viscosity, and suspend solids.

To effectively disperse and hydrate barley betafiber, the following techniques are suggested:
∘ Premix barley betafiber with other dry ingredients such as maltodextrin to help separate the particles during dispersion. Pre-blending barley betafiber in vegetable oil, corn syrup, or another nonsolvent may also be beneficial.
   - Preheat the water phase to 90°C prior to dispersing the barley betafiber.
   - Slowly meter the barley betafiber into the vortex of vigorously agitated hot water to thoroughly disperse. This can be achieved using high shear mixing. An aspirator apparatus such as a dispersion funnel and mixing eductor may also be useful to achieve good dispersion.
   - Allow the solution to hydrate and solubilize while mixing for 5-30 minutes. The required mixing time varies depending on the individual formula, process, and equipment used.

Potential applications for barley betafiber include: cream soups, clear soups, sauces, dips, dressings, spreads, and more.

Following is a condensed cream of chicken soup formula for heart health. It is low in fat and sodium, and contains 0.75g barley betafiber per 8oz serving (after 1:1 dilution with water).

**Condensed Soup for Heart Health**

| **Ingredients** | **%** |
|---|---|
| Water | 76.055 |
| Chicken Broth | 5.000 |
| Cargill Maltodextrin | 2.900 |
| Chicken Soup Base | 5.200 |
| Cargill Modified Food Starch | 3.200 |
| Precooked Chicken Cubes | 2.000 |
| Cargill Barley Betafiber | 1.200 |
| Whey Protein Concentrate | 1.000 |
| Cargill Soybean Salad Oil | 1.000 |
| Mono- and Diglycerides | 0.500 |
| Flavors | 0.800 |
| Spices and Seasoning | 0.970 |
| Microcrystalline Cellulose | 0.150 |
| Color | 0.025 |
| Total | 100.00 |

### Procedure:

1. Premix barley betafiber and maltodextrin then slowly add to the water while mixing with high shear. Blend for 3 minutes.
2. Add cellulose. Blend for 2 minutes.
3. Melt emulsifier in soybean oil and add to barley betafiber preparation with mixing.
4. Add starch and whey protein concentrate while mixing.
5. Premix seasonings, spices, and color. Add while mixing.
6. Add chicken base.
7. Blend the mixture for 3 minutes to obtain a smooth consistency.
8. Stir in chicken meat.
9. Fill cans or jars and retort.

### Example 22

A high purity (≥70% purity) ß-glucan product in accordance with the present invention, e.g. the barley betafiber described in Example 11 and Table 2, can be used in yogurt and other dairy applications for its functional and health benefits. The barley betafiber may also be used to build viscosity and impart a creamy mouthfeel. Barley betafiber is a versatile ingredient that may be incorporated into either the cultured milk phase or into a fruit flavor system that is blended with the yogurt.

The following formulation is for a yogurt containing 0.75g of barley β-glucan per 170g (6oz) serving.

**Plain Yogurt**

| **Ingredient** | **%** |
|---|---|
| Skim Milk | 86.35 |
| Cargill Sugar | 8.00 |
| Nonfat Dry Milk Solids | 3.00 |
| Stabilizer | 1.50 |
| Cargill Barley Betafiber | 0.65 |
| Culture | 0.50 |
| Total | 100.00 |

### Procedure:

1. Pre-blend the sugar, nonfat dry milk, stabilizer, and barley betafiber.
2. Blend dry ingredients into milk for 5 minutes until well hydrated.
3. Batch pasteurize at 85-88°C (185-190° F) over steam for 10 minutes.
4. Cool to 44°C (112° F) and whisk in starter culture.
5. Incubate to pH 4.6.
6. Blend until smooth and refrigerate to set.

Barley betafiber may also be delivered via a fruit flavoring system that is blended into the yogurt. The following fruit flavoring system is blended 80:20 yogurt to flavor system and will provide 0.75g Barley betafiber per 170g (6 oz) serving.

**Fruit Flavoring System**

| **Ingredient** | **%** |
|---|---|
| Liquid Sucrose (67% sugar) | 51.91 |
| Cargill High Fructose Corn Syrup | 28.75 |
| Fruit Pieces | 15.00 |
| Cargill Barley Betafiber | 3.24 |
| Color | 0.50 |
| Flavor | 0.60 |
| Total | 100.00 |

1. Premix sweeteners, barley betafiber, color and flavor.
2. Combine flavoring system with cultured yogurt at 20:80 ratio and blend until smooth.
3. Stir in fruit pieces.
4. Refrigerate to set.

### Example 23

Standardized Sensory Evaluation For Taste. A group of experienced panelists (n=4) are asked to taste a room temperature sample. The panelists are asked to come to a consensus on the overall flavor intensity of the sample, as well provide comments regarding the character of the flavor of the sample. The overall flavor intensity is rated on a scale of 0 to 7, where 0=bland, 1=threshold, 2=very slight, 3=slight, 4=slight moderate, 5=moderate, 6=moderate strong, and 7=strong. Comments on the character of the flavor can include cereal, green, woody, oxidized, bitter, etc.

In a specific case, four panelists were given a 1% by weight solution of a fiber composition according to the invention. The fiber composition had a purity of about 70% (i.e. the composition comprised about 70% barley betafiber) of a β-glucan fiber having a weight average molecular weight of about 185,000 and a viscosity of 57 cps. The panelists tasted the product at room temperature and together agreed that the over flavor intensity was a 4, and noted that the character of the flavor was "green" and "oatmeal."

## Claims

1. A dietary fiber composition isolated from a cereal grain containing β-glucan, comprising:
a β-glucan compound having a weight average molecular weight ranging from 120 kDa to 250 kDa, wherein said β-glucan compound is a modified form of a cereal β-glucan, and said β-glucan compound comprises at least 60% of the dietary fiber composition by weight; and,
wherein a 1% mixture by weight of said dietary fiber composition and water is stable and has a viscosity of about 100 cps or less.

2. A dietary fiber composition according to claim 1, wherein said dietary fiber has a protein content of less than about 3% by weight.

3. A dietary fiber composition according to claim 1, wherein said β-glucan compound comprises at least 70% of the composition by weight.

4. A dietary fiber composition according to claim 1, wherein the weight average molecular weight ranges from 120 kDa to 170 kDa.

5. A dietary fiber composition according to claim 1, wherein the viscosity is about 60 cps or less.

6. A dietary fiber composition according to claim 5, wherein the viscosity is about 5 or less.

7. A dietary fiber composition according to claim 1, wherein said dietary fiber composition has a fat content of about 2% or less.

8. A dietary fiber composition according to claim 1, wherein the composition is incorporated into a food or beverage product.

9. A dietary fiber composition according to claim 8, wherein the food or beverage product is a food product chosen from baked goods, cereal, extruded snacks, meat substitutes, bars, salad dressings, soup, sauces, yogurt, frozen desserts, refrigerated and frozen doughs, and confections.

10. A dietary fiber composition according to claim 9, wherein the food or beverage product is a baked good chosen from breads, rolls, buns, corn bread, quick breads, doughnuts, muffins, bagels, flatbreads, pancakes, waffles, cookies, cakes, pastries, croissants, scones, biscuits, crackers, pretzels, tortillas, taco shells, pasta, pie crusts, pizza crusts, and bakery mixes.

11. A dietary fiber composition according to claim 10, wherein the baked good is a white bread further comprising bread flour, all-purpose shortening, sugar, salt, calcium sulfate, dough conditioner, yeast, water and wheat gluten.

12. A dietary fiber composition according to claim 9, wherein the food or beverage product is a bar chosen from meal replacement bars, energy bars, high protein bars, granola bars, unfilled cereal bars, and filled cereal bars.

13. A dietary fiber composition according to claim 12, wherein the bar is a meal replacement bar further comprising soy protein, calcium caseinate, whey protein concentrate, vitamin and mineral premix, salt, high fructose corn syrup, high maltose corn syrup, honey, canola oil, soybean oil, and water.

14. A dietary fiber composition according to claim 8, wherein the food or beverage product is a soup further comprising water, chicken broth, maltodextrin, soup base, modified food starch, whey protein concentrate, soybean salad oil, mono- and diglycerides, and water.

15. A dietary fiber composition according to claim 8, wherein the food or beverage product is a yogurt, further comprising liquid sucrose, and high fructose corn syrup.

16. A dietary fiber composition according to claim 8, wherein the food or beverage product is a beverage product chosen from juice, juice drinks, milk, milk drinks, meal replacement beverages, diet and weight control beverages, powdered drink mixes, dairy-based drinks, dairy and non-dairy creamers, soy-based and rice-based beverages, energy and sports drinks, high-protein drinks, carbonated drinks, gel drinks, water and near water, tea-based beverages, coffee-based beverages, fruit and vegetable-based drinks, and smoothies.

17. A dietary fiber composition according to claim 16, wherein the food product is a juice drink further comprising fruit juice concentrate, high intensity sweeteners, acidulant, and water.

18. A composition, comprising: a dietary fiber composition in amount sufficient to lower LDL-C, wherein said dietary fiber composition comprises a β-glucan compound having a weight average molecular weight ranging from 120 kDa to 250 kDa, and said β-glucan compound comprises at least 60% of said dietary fiber composition by weight; and wherein a 1 % mixture by weight of said dietary fiber composition and water is stable and has a viscosity of about 1500 cps or less.

19. A method for obtaining a dietary fiber composition of any of claims 1-17, comprising:
forming an aqueous mixture having components which comprise a first exogenous enzyme, a second exogenous enzyme, and one or more cereal grains; wherein the one or more cereal grains comprise β-glucan and starch;
cleaving by a first hydrolysis reaction catalyzed by the first exogenous enzyme at least some of the bonds of the β-glucan, wherein the average molecular weight of the β-glucan is reduced; and hydrolysis of the starch by a second hydrolysis reaction catalyzed by the second exogenous enzyme, wherein the aqueous mixture has a temperature, and the method further comprises raising the temperature of the aqueous mixture to a level sufficiently high to substantially inactivate the first exogenous enzyme; adding to the aqueous mixture a third exogenous enzyme; and, cleaving by a third hydrolysis reaction catalyzed by at least the third exogenous enzyme at least some of the remaining uncleaved bonds of the starch, wherein the starch is substantially completely digested, wherein the third exogenous enzyme can be the same as the second exogenous enzyme when at least a portion of the first hydrolysis reaction and a portion of the second hydrolysis reaction occur substantially simultaneously;
separating and isolating a portion of the mixture, wherein the separated portion contains at least some of the β-glucan;
purifying the β-glucan within the separated portion;
obtaining a dietary fiber composition.

20. A method according to claim 19, wherein at least a portion of the first hydrolysis reaction and a portion of the second hydrolysis reaction occur substantially simultaneously.

21. A method according to claim 19, wherein the first exogenous enzyme also cleaves starch bonds.

22. A method according to claim 19, wherein the second hydrolysis occurs after the first hydrolysis, and the aqueous mixture has a temperature during the first hydrolysis, and the temperature is raised to a level sufficiently high to substantially inactivate the first exogenous enzyme prior to beginning the second hydrolysis.

23. A method according to claim 19, wherein the one or more cereal grains are selected from the group consisting of oat, barley, rye, and triticale.

24. A method according to claim 19, wherein the first exogenous enzyme exhibits cellulase activity.

25. A method according to claim 24, wherein the first exogenous enzyme further exhibits β-glucanase activity.

26. A method according to claim 24, wherein the first exogenous enzyme is active at temperatures above a starch gelatinization temperature.

27. A method according to claim 19, wherein the second exogenous enzyme exhibits amylolytic activity.

28. A method according to claim 19, wherein the aqueous mixture is an aqueous slurry.

29. A method according to claim 19, wherein the cleaving by the first hydrolysis reaction catalyzed by the first exogenous enzyme of at least some of the bonds of the β-glucan occurs while the temperature of the aqueous mixture is between 65 degrees C and 75 degrees C.

30. A method according to claim 22, wherein the hydrolysis of the starch by the second hydrolysis reaction catalyzed by the second exogenous enzyme occurs while the temperature of the aqueous mixture is between 90 degrees C and 110 degrees C, wherein the starch hydrolysis occurs until its digestion is substantially complete.

31. A method according to claim 19, wherein the dietary fiber composition includes about 1% fat or less.

32. A method according to claim 19, wherein the dietary fiber composition includes about 5% protein or less.

33. A method according to claim 19, wherein the dietary fiber composition includes about 75% dietary fiber or more.

34. A method according to claim 19, wherein the dietary fiber composition has a neutral mouthfeel.

## Patentansprüche

1. Ballaststoffzusammensetzung isoliert aus einem Getreidekorn, enthaltend βGlucan, umfassend:
eine β-Glucan-Verbindung mit einem gewichtsgemittelten Molekulargewicht im Bereich von 120 kDa bis 250 kDa, wobei die β-Glucan-Verbindung eine modifizierte Form eines Getreide-β-Glucans ist, und die β-Glucan-Verbindung mindestens 60 % der Ballaststoffzusammensetzung, bezogen auf das Gewicht,
umfasst; und
wobei eine 1 % Mischung, bezogen auf das Gewicht, der Ballaststoffzusammensetzung und Wasser stabil ist und eine Viskosität von etwa 100 cps oder
weniger aufweist.

2. Ballaststoffzusammensetzung nach Anspruch 1, wobei die Ballaststoffe einen Proteingehalt von weniger als etwa 3 %, bezogen auf das Gewicht, aufweisen.

3. Ballaststoffzusammensetzung nach Anspruch 1, wobei die β-Glucan-Verbindung mindestens 70 % der Zusammensetzung, bezogen auf das Gewicht, umfasst.

4. Ballaststoffzusammensetzung nach Anspruch 1, wobei das gewichtsgemittelte Molekulargewicht von 120 kDa bis 170 kDa reicht.

5. Ballaststoffzusammensetzung nach Anspruch 1, wobei die Viskosität etwa 60 cps oder weniger beträgt.

6. Ballaststoffzusammensetzung nach Anspruch 5, wobei die Viskosität etwa 5 oder weniger beträgt.

7. Ballaststoffzusammensetzung nach Anspruch 1, wobei die Ballaststoffzusammensetzung einen Fettgehalt von etwa 2 % oder weniger aufweist.

8. Ballaststoffzusammensetzung nach Anspruch 1, wobei die Zusammensetzung in ein Lebensmittel oder Getränkeprodukt inkorporiert ist.

9. Ballaststoffzusammensetzung nach Anspruch 8, wobei das Lebensmittel oder Getränkeprodukt ein Lebensmittelprodukt ist, ausgewählt aus Backwaren, Cerealien, extrudierten Snacks, Fleischersatzprodukten, Riegeln, Salatdressings, Suppe, Soßen, Joghurt, gefrorenen Desserts, gekühlten und gefrorenen Teigen und Konfekten.

10. Ballaststoffzusammensetzung nach Anspruch 9, wobei das Lebensmittel oder Getränkeprodukt eine Backware ist, ausgewählt aus Broten, Semmeln, Brötchen, Maisbrot, Schnellbroten, Donuts, Muffins, Bagles, Flachbroten, Pfannkuchen, Waffeln, Keksen, Kuchen, Gebäck, Croissants, Scones, Biscuits, Cracker, Brezeln, Tortilla, Taco shells, Pasta, Tortenböden, Pizzaböden und Bäckereimischungen.

11. Ballaststoffzusammensetzung nach Anspruch 10, wobei die Backware ein Weißbrot ist, des Weiteren umfassend Brotmehl, Allzweckfett, Zucker, Salz, Calciumsulfat, Teigkonditionierer, Hefe, Wasser und Weizengluten.

12. Ballaststoffzusammensetzung nach Anspruch 9, wobei das Lebensmittel oder das Getränkeprodukt ein Riegel ist, ausgewählt aus Mahlzeitersatzriegeln, Energieriegeln, Hochproteinriegeln, Müsliriegeln, ungefüllten Cerealienriegeln und gefüllten Cerealienriegeln.

13. Ballaststoffzusammensetzung nach Anspruch 12, wobei der Riegel ein Mahlzeitersatzriegel ist, des Weiteren umfassend Sojaprotein, Calciumcaseinat, Molkeproteinkonzentrat, Vitamin und Mineralvormischung, Salz, Maissirup mit hohem Fruktosegehalt, Maissirup mit hohem Maltosegehalt, Honig, Canolaöl, Sojabohnenöl und Wasser.

14. Ballaststoffzusammensetzung nach Anspruch 8, wobei das Lebensmittel oder Getränkeprodukt eine Suppe ist, des Weiteren umfassend Wasser, Hühnerbrühe, Maltodextrin, Suppenbasis, modifizierte Lebensmittelstärke, Molkeproteinkonzentrat, Sojabohnensalatöl, Mono- und Diglyceride und Wasser.

15. Ballaststoffzusammensetzung nach Anspruch 8, wobei das Lebensmittel oder Getränkeprodukt ein Joghurt ist, des Weiteren umfassend flüssige Saccharose und Maissirup mit hohem Fruktosegehalt.

16. Ballaststoffzusammensetzung nach Anspruch 8, wobei das Lebensmittel oder Getränkeprodukt ein Getränkeprodukt ist, ausgewählt aus Saft, Saftgetränken, Milch, Milchgetränken, Mahlzeitersatzgetränken, Diät- und Gewichtskontrollgetränken, pulvrigen Getränkemischungen, Milch-basierten Getränken, Milch und Nicht-Milch-Kaffeesahne, Soja-basierten und Reis-basierten Getränken, Energie- und Sportgetränken, Getränken mit hohem Proteingehalt, kohlensäurehaltigen Getränken, Gelgetränken, Wasser und Near-Water, Tee-basierten Getränken, Kaffee-basierten Getränken, Frucht- und Gemüse-basierten Getränken und Smoothies.

17. Ballaststoffzusammensetzung nach Anspruch 16, wobei das Lebensmittelprodukt ein Fruchtgetränk ist, des Weiteren umfassend Fruchtsaftkonzentrat, Süßstoffe mit hoher Intensität, Säuerungsmittel und Wasser.

18. Zusammensetzung, umfassend: eine Ballaststoffzusammensetzung in einer Menge, ausreichend, um LDL-C zu senken, wobei die Ballaststoffzusammensetzung eine β-Glucan-Verbindung mit einem gewichtsgemittelten Molekulargewicht im Bereich von 120 kDa bis 250 kDa umfasst, und die β-Glucan-Verbindung mindestens 60 % der Ballaststoffzusammensetzung, bezogen auf das Gewicht, umfasst; und wobei eine 1 %-Mischung, bezogen auf das Gewicht, der Ballaststoffzusammensetzung und Wasser stabil ist und eine Viskosität von etwa 1500 cps oder weniger aufweist.

19. Verfahren zum Erhalten einer Ballaststoffzusammensetzung nach einem beliebigen der Ansprüche 1-17, umfassend:
Bilden einer wässrigen Mischung mit Komponenten, die ein erstes exogenes Enzym, ein zweites exogenes Enzym und ein oder mehrere Getreidekörner umfasst; wobei das eine oder die mehreren Getreidekörner β-Glucan und
Stärke umfasst/umfassen;
Spalten durch eine erste Hydrolysereaktion, katalysiert durch das erste exogene Enzym, mindestens einiger der Bindungen des β-Glucans, wobei das gemittelte Molekulargewicht des β-Glucans reduziert wird; und Hydrolyse der Stärke durch eine zweite Hydrolysereaktion, katalysiert durch das zweite exogene Enzym, wobei die wässrige Mischung eine Temperatur aufweist, und das Verfahren des Weiteren das Anheben der Temperatur der wässrigen Mischung auf einen Spiegel umfasst, der ausreichend hoch ist, das erste exogene Enzym im Wesentlichen zu inaktivieren; Hinzufügen eines dritten exogenen Enzyms zu dem wässrigen Gemisch; und Spalten durch eine dritte Hydrolysereaktion, katalysiert durch mindestens das dritte exogene Enzym mindestens einiger der verbleibenden ungespaltenen Bindungen der Stärke, wobei die Stärke im Wesentlichen vollständig verdaut ist, wobei das dritte exogene Enzym das gleiche wie das zweite exogene Enzym sein kann, wenn mindestens ein Teil der ersten Hydrolysereaktion und ein Teil der zweiten Hydrolysereaktion im Wesentlichen gleichzeitig stattfinden;
Trennen und Isolieren eines Teils des Gemisches, wobei der getrennte Teil mindestens etwas des β-Glucans enthält;
Reinigen des β-Glucans innerhalb des abgetrennten Teils;
Erhalten einer Ballaststoffzusammensetzung.

20. Verfahren nach Anspruch 19, wobei mindestens ein Teil der ersten Hydrolysereaktion und ein Teil der zweiten Hydrolysereaktion im Wesentlichen gleichzeitig stattfinden.

21. Verfahren nach Anspruch 19, wobei das erste exogene Enzym auch Stärkebindungen spaltet.

22. Verfahren nach Anspruch 19, wobei die zweite Hydrolyse nach der ersten Hydrolyse stattfindet und das wässrige Gemisch eine Temperatur während der ersten Hydrolyse aufweist, und die Temperatur auf einen Spiegel angehoben wird, ausreichend hoch, um das erste exogene Enzym vor dem Beginnen der zweiten Hydrolyse im Wesentlichen zu inaktivieren.

23. Verfahren nach Anspruch 19, wobei das eine oder die mehreren Getreidekorn/ Getreidekörner ausgewählt sind aus der Gruppe, bestehend aus Hafer, Gerste, Roggen und Triticale.

24. Verfahren nach Anspruch 19, wobei das erste exogene Enzym Cellulase-Aktivität aufweist.

25. Verfahren nach Anspruch 24, wobei das erste exogene Enzym des Weiteren β-Glucanase-Aktivität aufweist.

26. Verfahren nach Anspruch 24, wobei das erste exogene Enzym bei Temperaturen oberhalb einer Stärkegelierungstemperatur aktiv ist.

27. Verfahren nach Anspruch 19, wobei das zweite exogene Enzym amylolytische Aktivität aufweist.

28. Verfahren nach Anspruch 19, wobei die wässrige Mischung eine wässrige Aufschlämmung ist.

29. Verfahren nach Anspruch 19, wobei das Spalten durch die erste Hydrolysereaktion, katalysiert durch das erste exogene Enzym, von mindestens einiger der Bindungen des β-Glucans stattfindet, während die Temperatur der wässrigen Mischung zwischen 65 °C und 75 °C ist.

30. Verfahren nach Anspruch 22, wobei die Hydrolyse der Stärke durch die zweite Hydrolysereaktion, katalysiert durch das zweite exogene Enzym, stattfindet, während die Temperatur der wässrigen Mischung zwischen 90 °C und 110 °C ist, wobei die Stärkehydrolyse stattfindet, bis dessen Verdau im Wesentlichen vollständig ist.

31. Verfahren nach Anspruch 19, wobei die Ballaststoffzusammensetzung etwa 1 % Fett oder weniger einschließt.

32. Verfahren nach Anspruch 19, wobei die Ballaststoffzusammensetzung etwa 5 % Protein oder weniger einschließt.

33. Verfahren nach Anspruch 19, wobei die Ballaststoffzusammensetzung etwa 75 % Ballaststoffe oder mehr einschließt.

34. Verfahren nach Anspruch 19, wobei die Ballaststoffzusammensetzung ein neutrales Mundgefühl aufweist.

## Revendications

1. Composition de fibres diététiques isolées à partir d'un grain de céréale contenant du β-glucane, comprenant:
un composé β-glucane ayant un poids moléculaire moyen en poids allant de 120 kDa à 250 kDa, dans lequel ledit composé β-glucane est une forme modifiée d'un β-glucane de céréale, et ledit composé β-glucane constitue au moins 60 % de la composition de fibres diététiques en poids; et,
dans laquelle un mélange à 1 % en poids de ladite composition de fibres diététiques et d'eau est stable et a une viscosité d'environ 100 cps ou moins.

2. Composition de fibres diététiques selon la revendication 1, dans laquelle ladite fibre diététique a une teneur en protéines inférieure à environ 3 % en poids.

3. Composition de fibres diététiques selon la revendication 1, dans laquelle ledit composé β-glucane constitue au moins 70 % de la composition en poids.

4. Composition de fibres diététiques selon la revendication 1, dans laquelle le poids moléculaire moyen en poids va de 120 kDa à 170 kDa.

5. Composition de fibres diététiques selon la revendication 1, dans laquelle la viscosité est d'environ 60 cps ou moins.

6. Composition de fibres diététiques selon la revendication 5, dans laquelle la viscosité est d'environ 5 ou moins.

7. Composition de fibres diététiques selon la revendication 1, dans laquelle ladite composition de fibres diététiques a une teneur en matières grasses d'environ 2 % ou moins.

8. Composition de fibres diététiques selon la revendication 1, dans laquelle la composition est incorporée dans un produit alimentaire ou une boisson.

9. Composition de fibres alimentaires selon la revendication 8, dans laquelle le produit alimentaire ou la boisson est un produit alimentaire choisi parmi les produits de boulangerie-pâtisserie, les céréales, les grignotines extrudées, les substituts de viande, les barres, les vinaigrettes, les soupes, les sauces, les yaourts, les desserts congelés, les pâtes réfrigérées et surgelées, et les confiseries.

10. Composition de fibres diététiques selon la revendication 9, dans laquelle le produit alimentaire ou la boisson est un produit de boulangerie-pâtisserie choisi parmi les pains, les roulés, les petits pains au lait, le pain de maïs, les pains express, les beignets, les muffins, les bagels, les pains plats, les crêpes, les gaufres, les cookies, les gâteaux, les pâtisseries, les croissants, les scones, les biscuits, les craquelins, les bretzels, les tortillas, les tacos, les pâtes, les pâtes à tarte, les pâtes à pizza, et les mélanges de boulangerie.

11. Composition de fibres diététiques selon la revendication 10, dans laquelle le produit de boulangerie-pâtisserie est un pain blanc comprenant en outre de la farine pour pain, du shortening tout usage, du sucre, du sel, du sulfate de calcium, un conditionneur de pâte, de la levure, de l'eau et du gluten de blé.

12. Composition de fibres alimentaires selon la revendication 9, dans laquelle le produit alimentaire ou la boisson est une barre choisie parmi les barres substituts de repas, les barres énergétiques, les barres riches en protéines, les barres de céréales, les barres de céréales non fourrées et les barres de céréales fourrées.

13. Composition de fibres diététiques selon la revendication 12, dans laquelle la barre est une barre substitut de repas comprenant en outre une protéine de soja, du caséinate de calcium, un concentré de protéine de lactosérum, un prémélange de vitamines et minéraux, du sel, un sirop de maïs à teneur élevée en fructose, un sirop de maïs à teneur élevée en maltose, du miel, de l'huile de canola, de l'huile de soja et de l'eau.

14. Composition de fibres diététiques selon la revendication 8, dans laquelle le produit alimentaire ou la boisson est une soupe comprenant en outre de l'eau, du bouillon de poulet, de la maltodextrine, une préparation pour soupe, un amidon alimentaire modifié, un concentré de protéine de lactosérum, une huile de salade de soja, des mono et diglycérides et de l'eau.

15. Composition de fibres diététiques selon la revendication 8, dans laquelle le produit alimentaire ou la boisson est un yaourt, comprenant en outre du saccharose liquide, et un sirop de maïs à teneur élevée en fructose.

16. Composition de fibres diététiques selon la revendication 8, dans laquelle le produit alimentaire ou la boisson est une boisson choisie parmi un jus, les boissons à base de jus, le lait, les boissons à base de lait, les boissons substituts de repas, les boissons diététiques et de contrôle du poids, les préparations pour boissons en poudre, les boissons à base de produits laitiers, les crèmes à café laitières et non laitières, les boissons à base de soja et à base de riz, les boissons énergétiques et pour sportifs, les boissons riches en protéines, les boissons gazeuses, les boissons gélifiées, l'eau et l'eau à valeur ajoutée, les boissons à base de thé, les boissons à base de café, les boissons à base de fruits et de légumes et les boissons fouettées.

17. Composition de fibres diététiques selon la revendication 16, dans laquelle le produit alimentaire est une boisson à base de jus comprenant en outre un concentré de jus de fruits, des édulcorants de haute intensité, un acidifiant et de l'eau.

18. Composition, comprenant : une composition de fibres diététiques dans une quantité suffisante pour abaisser le LDL-C, dans laquelle ladite composition de fibres diététiques comprend un composé β-glucane ayant un poids moléculaire moyen en poids allant de 120 kDa à 250 kDa, et ledit composé β-glucane constitue au moins 60 % de ladite composition de fibres diététiques en poids ; et dans laquelle un mélange à 1 % en poids de ladite composition de fibres diététiques et d'eau est stable et a une viscosité d'environ 1 500 cps ou moins.

19. Procédé pour obtenir une composition de fibres diététiques selon l'une quelconque des revendications 1 à 17, comprenant:
la formation d'un mélange aqueux ayant des composants qui comprennent une première enzyme exogène, une deuxième enzyme exogène, et un ou plusieurs grains de céréales ; dans lequel les un ou plusieurs grains de céréales comprennent un β-glucane et de l'amidon;
le clivage par une première réaction d'hydrolyse catalysée par la première enzyme exogène d'au moins certaines des liaisons du β-glucane, dans lequel le poids moléculaire moyen du β-glucane est réduit ; et l'hydrolyse de l'amidon par une deuxième réaction d'hydrolyse catalysée par la deuxième enzyme exogène, dans lequel le mélange aqueux a une température, et le procédé comprend en outre l'élévation de la température du mélange aqueux à un niveau suffisamment élevé pour inactiver sensiblement la première enzyme exogène ; l'ajout au mélange aqueux d'une troisième enzyme exogène ; et, le clivage par une troisième réaction d'hydrolyse catalysée par au moins la troisième enzyme exogène d'au moins certaines des liaisons non clivées restantes de l'amidon, dans lequel l'amidon est digéré sensiblement complètement, dans lequel la troisième enzyme exogène peut être identique à la deuxième enzyme exogène quand au moins une partie de la première réaction d'hydrolyse et une partie de la deuxième réaction d'hydrolyse ont lieu sensiblement simultanément;
la séparation et l'isolement d'une partie du mélange, dans lequel la partie séparée contient au moins une partie du β-glucane;
la purification du β-glucane au sein de la partie séparée;
l'obtention d'une composition de fibres diététiques.

20. Procédé selon la revendication 19, dans lequel au moins une partie de la première réaction d'hydrolyse et une partie de la deuxième réaction d'hydrolyse ont lieu sensiblement simultanément.

21. Procédé selon la revendication 19, dans lequel la première enzyme exogène clive également les liaisons de l'amidon.

22. Procédé selon la revendication 19, dans lequel la deuxième hydrolyse a lieu après la première hydrolyse, et le mélange aqueux a une température au cours de la première hydrolyse, et la température est élevée à un niveau suffisamment élevé pour inactiver sensiblement la première enzyme exogène avant le début de la deuxième hydrolyse.

23. Procédé selon la revendication 19, dans lequel les un ou plusieurs grains de céréales sont choisis dans le groupe constitué par l'avoine, l'orge, le seigle et le triticale.

24. Procédé selon la revendication 19, dans lequel la première enzyme exogène présente une activité cellulase.

25. Procédé selon la revendication 24, dans lequel la première enzyme exogène présente en outre une activité β-glucanase.

26. Procédé selon la revendication 24, dans lequel la première enzyme exogène est active à des températures supérieures à une température de gélatinisation de l'amidon.

27. Procédé selon la revendication 19, dans lequel la deuxième enzyme exogène présente une activité amylolytique.

28. Procédé selon la revendication 19, dans lequel le mélange aqueux est une suspension aqueuse.

29. Procédé selon la revendication 19, dans lequel le clivage par la première réaction d'hydrolyse catalysée par la première enzyme exogène d'au moins certaines des liaisons du β-glucane a lieu tandis que la température du mélange aqueux est comprise entre 65 degrés C et 75 degrés C.

30. Procédé selon la revendication 22, dans lequel l'hydrolyse de l'amidon par la deuxième réaction d'hydrolyse catalysée par la deuxième enzyme exogène a lieu tandis que la température du mélange aqueux est comprise entre 90 degrés C et 110 degrés C, dans lequel l'hydrolyse de l'amidon a lieu jusqu'à ce que sa digestion soit sensiblement complète.

31. Procédé selon la revendication 19, dans lequel la composition de fibres diététiques comprend environ 1 % de matières grasses ou moins.

32. Procédé selon la revendication 19, dans lequel la composition de fibres diététiques comprend environ 5 % de protéine ou moins.

33. Procédé selon la revendication 19, dans lequel la composition de fibres diététiques comprend environ 75 % de fibres diététiques ou plus.

34. Procédé selon la revendication 19, dans lequel la composition de fibres diététiques a une sensation en bouche neutre.
